# FASCICULE DE BREVET EUROPEEN

(11) **EP 2 046 716 B1**
(45) Date de publication et mention de la délivrance du brevet: **14.08.2013**
(21) Numéro de dépôt: 07786798.4
(22) Date de dépôt: 21.06.2007
(51) Int. Cl.: C07C 59/84, C07C 59/88, C07C 59/90, C07C 251/48, A61K 31/192, A61P 3/06, A61P 3/10, A61P 3/04, C07C 59/68, C07C 323/22, C07C 323/62, C07C 69/736, C07D 213/30

(54) **DERIVES DE 1,3-DIPHENYLPROPANE SUBSTITUES, PREPARATIONS ET UTILISATIONS**
SUBSTITUIERTE 1,3-DIPHENYLPROPANDERIVATE, HERSTELLUNG UND ANWENDUNGEN DAVON
SUBSTITUTED 1,3-DIPHENYLPROPANE DERIVATIVES, PREPARATIONS AND USES THEREOF

(30) Priorité: 21.06.2006 FR 0605540
(43) Date de publication de la demande: 15.04.2009
(73) Titulaire: Genfit, 59120 Loos (FR)
(72) Inventeur: DELHOMEL, Jean-François, F-62144 Acq (FR); HANF, Rémy, 59000 Lille (FR); CAUMONT-BERTRAND, Karine, 59236 Frelinghien (FR)
(74) Mandataire: Tezier Herman, Béatrice
(86) Numéro de dépôt international: PCT/EP2007/056225
(87) Numéro de publication internationale: WO 2007/147880

(56) Documents cités:
- DE-A1- 4 121 849
- DE-A1- 4 327 365
- MORISHITA S ET AL: "SYNTHESIS AND HYPOLILIDAEMIC ACTIVITY OF 2-SUBSTITUTED ISOBUTYRIC ACID DERIVATIVES" JOURNAL OF MEDICINAL CHEMISTRY, AMERICAN CHEMICAL SOCIETY. WASHINGTON, US, vol. 31, no. 6, juin 1988 (1988-06), pages 1205-1209, XP002003702 ISSN: 0022-2623
- LABAUDINIERE R ET AL: "OMEGA-[(OMEGA-ARYLALKYL)ARYL]ALKANOIC ACIDS: A NEW CLASS OF SPECIFIC LTA4 HYDROLASE INHIBITORS" JOURNAL OF MEDICINAL CHEMISTRY, AMERICAN CHEMICAL SOCIETY. WASHINGTON, US, vol. 35, no. 17, 1992, pages 3156-3169, XP001205195 ISSN: 0022-2623
- DATABASE CA [Online] CHEMICAL ABSTRACTS SERVICE, COLUMBUS, OHIO, US; HE, LAN ET AL: "Synthesis and biological activity of flavane derivatives" XP002425766 extrait de STN Database accession no. 2006:290724 & CHINESE JOURNAL OF CHEMISTRY , 24(3), 401-408 CODEN: CJOCEV; ISSN: 1001-604X, 2006,

## Description

La présente invention concerne des composés dérivés de 1,3-diphénylpropane substitués, les compositions pharmaceutiques les comprenant ainsi que leurs applications thérapeutiques, notamment dans les domaines de la santé humaine et animale.

Les inventeurs ont mis en évidence, de manière surprenante, que les composés selon l'invention possèdent de manière intrinsèque des propriétés agonistes PPAR (Peroxisome Proliferator-Activated Receptor).

Les molécules décrites dans l'invention sont donc d'un intérêt particulier pour traiter les complications associées au syndrome métabolique, l'insulino-résistance, le diabète, les dyslipidémies, l'athérosclérose, les maladies cardiovasculaires, l'obésité, l'hypertension, les maladies inflammatoires (asthme, etc.), les pathologies neurodégénératives (Alzheimer, etc.), les cancers, etc., ainsi que pour permettre la diminution du risque global. Préférentiellement, les composés selon l'invention sont utilisables pour le traitement des dyslipidémies.

Le diabète, l'obésité et les dyslipidémies (taux plasmatiques de cholestérol LDL et de triglycérides élevés, cholestérol HDL faible, etc.) font partie des facteurs de risque cardiovasculaire clairement identifiés qui prédisposent un individu à développer une pathologie cardiovasculaire (Mensah M, 2004). Ces facteurs de risque s'additionnent aux facteurs de risque liés au mode de vie tels que le tabagisme, l'inactivité physique et les régimes alimentaires déséquilibrés. Un effet synergique existe entre ces différents facteurs : la présence concomitante de plusieurs d'entre eux conduit à une aggravation dramatique du risque cardiovasculaire et il convient alors de parler de risque global (« *global risk* ») pour les maladies cardiovasculaires. La prévalence des dyslipidémies atteignait 43,6% de la population en 2004 dans les principaux pays développés. La prévalence du diabète, actuellement en nette augmentation, est en passe de devenir de plus en plus significative dans l'épidémiologie des maladies cardiovasculaires : la prévalence du diabète est en effet estimée à 7,6% de la population pour 2010 (Fox-Tucker J, 2005).

Selon l'International Atherosclerosis Society (International Atherosclerosis Society, 2003), les maladies cardiovasculaires représentent la première cause de mortalité dans les pays industrialisés et deviennent de plus en plus fréquentes dans les pays en voie de développement. Ces maladies sont notamment les maladies coronariennes, l'ischémie cérébrale et les maladies artérielles périphériques.

Ces données justifient donc l'adoption de mesures énergiques pour réduire significativement la morbidité et la mortalité cardiovasculaires et la nécessité de trouver des traitements efficaces, complémentaires d'une modification de l'hygiène de vie, agissant sur les facteurs de risque des maladies cardiovasculaires et sur leurs conséquences devient une urgence mondiale.

Les composés selon l'invention, par leurs propriétés agonistes PPAR, présentent un intérêt particulier pour le traitement des pathologies liées aux dérèglements du métabolisme lipidique et/ou glucidique, telles que le diabète, l'obésité, les dyslipidémies ou l'inflammation, ainsi que pour la diminution du risque cardiovasculaire global.

Les PPAR (α, γ et δ) sont en effet connus comme étant impliqués dans ce type de pathologies (Kota BP *et al.,* 2005) : des ligands de ces récepteurs sont donc commercialisés pour traiter de telles pathologies (Lefebvre P *et al.,* 2006) et de nombreux modulateurs PPAR, agonistes ou antagonistes, sélectifs ou non, sont actuellement en développement avancé. Un modulateur PPAR ayant des effets bénéfiques sur la résistance à l'insuline, l'obésité, les dyslipidémies, l'hypertension et/ou l'inflammation pourrait être utilisé dans le traitement du syndrome métabolique (ou syndrome X) (Liu Y and Miller A, 2005).

La famille des PPAR comprend trois isoformes, désignés α, γ et δ (encore appelé β), chacun codé par un gène différent. Ces récepteurs font partie de la superfamille des récepteurs nucléaires et des facteurs de transcription qui sont activés par la liaison de certains acides gras et/ou de leurs métabolites lipidiques. Les PPAR activés forment des hétérodimères avec les récepteurs de l'acide rétinoïque 9-cis (RXR ou Retinoid X Receptor) et se fixent sur des éléments de réponse spécifiques (PPRE ou Peroxisome Proliferator Response Element) au niveau du promoteur de leurs gènes cibles, permettant ainsi le contrôle de la transcription.

PPARα contrôle le métabolisme lipidique (hépatique et musculaire) et l'homéostasie du glucose, influence le métabolisme intracellulaire des lipides et des sucres par un contrôle direct de la transcription de gènes codant pour des protéines impliquées dans l'homéostasie lipidique, exerce des effets anti-inflammatoires et anti-prolifératifs et prévient les effets pro-athérogéniques de l'accumulation du cholestérol dans les macrophages en stimulant l'efflux du cholestérol (Lefebvre P, Chinetti G, Fruchart JC and Staels B, 2006). Les fibrates (fénofibrate, bézafibrate, ciprofibrate, gemfibrozil), par l'intermédiaire de PPARα, sont ainsi utilisés en clinique dans le traitement de certaines dyslipidémies en baissant les triglycérides et en augmentant les taux de HDL (High Density Lipoprotein).

PPARγ est un régulateur-clé de l'adipogenèse. De plus, il est impliqué dans le métabolisme lipidique des adipocytes matures, dans l'homéostasie du glucose, notamment dans la résistance à l'insuline, dans l'inflammation, dans l'accumulation de cholestérol au niveau des macrophages et dans la prolifération cellulaire (Lehrke M and Lazar MA, 2005). PPARγ joue par conséquent un rôle dans la pathogenèse de l'obésité, de l'insulino-résistance et du diabète. Les thiazolidinediones (Rosiglitazone, Troglitazone, etc.) sont des ligands du récepteur PPARγ utilisés dans le traitement du diabète de type 2.

Il existe des ligands de PPARδ (L-165041, GW501516 actuellement en développement clinique) mais aucun ligand PPARδ n'est actuellement utilisé comme médicament. Ce récepteur est cependant une cible attractive pour le développement de médicaments utilisables dans le traitement des dyslipidémies, de l'athérosclérose, de l'obésité et de la résistance à l'insuline : PPARδ est en effet impliqué dans le contrôle du métabolisme lipidique et glucidique, dans la balance énergétique, dans la neurodégénération, dans l'obésité, dans la formation des cellules spumeuses et dans l'inflammation (Gross B *et al.,* 2005).

Au-delà du rôle direct joué par les ligands PPAR sur la régulation du métabolisme des lipides et des glucides, ces molécules ont un spectre d'action pléiotropique dû à la grande diversité des gènes cibles des PPAR. Ces multiples propriétés font des PPAR des cibles thérapeutiques d'intérêt pour le traitement de maladies comme l'athérosclérose, l'ischémie cérébrale, l'hypertension, les maladies liées à une néo-vascularisation (rétinopathies diabétiques, etc.), les maladies inflammatoires et auto-immunes (maladie de Crohn, psoriasis, sclérose en plaques, asthme, etc), les maladies néoplasiques (carcinogenèse, etc.), les maladies neurodégénératives, les complications associées au syndrome métabolique, l'insulino-résistance, le diabète, les dyslipidémies, les maladies cardiovasculaires, l'obésité, etc., ainsi que pour permettre la diminution du risque global.

Les composés selon l'invention, par leurs propriétés agonistes PPAR, représentent donc un outil thérapeutique avantageux pour l'amélioration des pathologies liées aux dérèglements du métabolisme lipidique et/ou glucidique, notamment des dyslipidémies, ainsi que pour la diminution du risque cardiovasculaire global.

Plus généralement, en agissant de manière simultanée sur plusieurs processus de régulation, les composés selon l'invention représentent un moyen thérapeutique avantageux pour traiter les complications associées au syndrome métabolique (dont les caractéristiques sont l'obésité, en particulier l'obésité abdominale, une concentration anormale de lipides sanguins (taux élevé de triglycérides et/ou faible taux de cholestérol HDL (dyslipidémie)), une glycémie élevée et/ou une résistance à l'insuline et une hypertension), l'athérosclérose, les maladies cardiovasculaires, l'insulino-résistance, l'obésité, l'hypertension, le diabète, les dyslipidémies, les maladies cardiovasculaires, les maladies inflammatoires (asthme, etc.), les pathologies neurodégénératives (Alzheimer, etc.), les cancers, etc., ainsi que pour permettre la diminution du risque global.

Morishita et al. (Morishita et al : « Synthesis and hypolipidaemic activity of 2-substituted isobutyric acid derivatives » Journal of Medicinal Chemistry, American Chemical Society. Washington, US, vol. 31, no. 6, juin 1988 (1988-06), pages 1205-1209) divulgue des composés hypolipémiants, differents de la présente invention. Les documents DE 41 21 849 et Labaudinère R. et al. (Journal of Medicinal Chemistry, American Chemical Society, Washington, US, vol. 35, no. 17, 1992, pages 3156-3169) décrivent des inhibiteurs de l'hydrolase du leukotriène A4 dans le traitement de l'inflammation chronique tel que le traitement du rhumatisme, psoriasis et autres maladies de la peau, mais les composés décrits sont différents de ceux de la présente invention. Il en est de même du document DE 46 27 365 qui divulgue des composés phénols et dérivés phénols comme agents diminuant le taux de fibrinogène plasmatique.

La présente invention a pour objet des composés dérivés de 1,3-diphénylpropane substitués de formule générale (I) suivante : 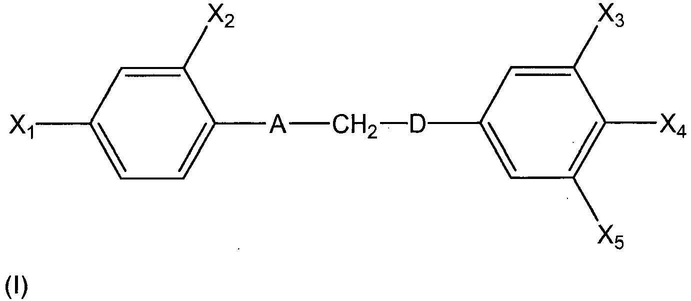 dans laquelle :
X1 représente un atome d'halogène, un groupement R1 ou G1-R1;
X2 représente un atome d'hydrogène;
X3 représente un groupement R3 ;
X4 représente G4-R4, dans lequel G4 représente un atome d'oxygène;
X5 représente un groupement R5 ;
R1 représentant un groupement alkyle halogéné;
R2 représentant un atome d'hydrogène ou un groupement alkyle;
R3 et R5, identiques ou différents, représentant un groupement alkyle non substitué;
R4 représentant un groupement alkyle substitué par un groupement COOR9;
G1 représentant un atome d'oxygène ou de soufre ;
A représente :
   (i) un groupement -CR6R7 dans lequel:
      R6 représente un atome d'hydrogène,
      et R7 représente un groupement hydroxy ou un groupement -OR8, R8 étant tel que défini ci-dessous,
   (ii) un groupement carbonyle (CO),
R8 représentant un groupement alkyle, substitué ou non par un groupement aryle ou cycloalkyle;
D représente un atome de carbone lié à deux atomes d'hydrogène (CH₂),
R9 représentant un atome d'hydrogène ou un radical alkyle non substitué;
leurs stéréoisomères (diastéréoisomères, énantiomères), purs ou en mélange, mélanges racémiques, isomères géométriques, tautomères, sels, hydrates, solvates, formes solides ainsi que leurs mélanges.

Dans le cadre de la présente invention, le terme « alkyle » désigne un radical hydrocarboné saturé, linéaire, ramifié ou cyclique, halogéné ou non, ayant plus particulièrement de 1 à 24, de préférence 1, 2, 3, 4, 5, 6, 7, 8, 9 ou 10, atomes de carbone. On peut citer, par exemple, les radicaux méthyle, trifluorométhyle, éthyle, n-propyle, isopropyle, n-butyle, isobutyle, tertiobutyle, sec-butyle, pentyle, néopentyle, n-hexyle ou cyclohexyle.

Le terme « cycloalkyle » désigne un groupe alkyle tel que défini ci-dessus et formant au moins un cycle. On peut citer à titre de groupes cycloalkyle ayant de 3 à 8 atomes de carbone, le cyclopropyle, cyclobutyle, cyclopentyle, cyclohexyle, cycloheptyle et cyclooctyle.

Le terme « alkyloxy » fait référence à une chaîne alkyle liée à la molécule par l'intermédiaire d'un atome d'oxygène (liaison éther). La chaîne alkyle répond à la définition précédemment énoncée. A titre d'exemple, on peut citer les radicaux méthoxy, trifluorométhoxy, éthoxy, n-propyloxy, isopropyloxy, n-butoxy, iso-butoxy, tertio-butoxy, sec-butoxy ou hexyloxy.

Le terme « aryle » fait référence à des groupes aromatiques comprenant de préférence 5 à 14 atomes de carbone, avantageusement 6 à 14 atomes de carbone, éventuellement interrompus par un ou plusieurs hétéroatomes choisis parmi N, O, S ou P (nommés plus spécifiquement « hétéroaryle »). Ils sont généralement mono- ou bicycliques et comprennent avantageusement de 6 à 14 atomes de carbone, tels que le phényle, α-naphtyle, β-naphtyle, anthracényle ou fluorényle.

Le terme « hétérocycle oxygéné ou soufré » désigne un groupe cycloalkyle tel que défini ci-dessus interrompu par un ou plusieurs hétéroatomes choisis parmi O et S. On peut ainsi citer à titre d'exemple le thiopyrane ou pyrane.

Par atome d'halogène, on entend un atome de brome, chlore, fluor ou iode.

Un radical alkyle halogéné est un radical alkyle tel que défini ci-dessus présentant au moins un atome d'halogène, voire totalement halogéné (perhalogéné).

Un aspect particulier de l'invention concerne les composés de formule générale (I) dans laquelle A représente un groupement carbonyle (CO).

Un autre aspect particulier de l'invention concerne les composés de formule générale (I) dans laquelle A représente un groupement -CR6R7, R6 représentant un atome d'hydrogène et R7 représentant un groupement hydroxy.

Un autre aspect préféré de l'invention concerne les composés de formule générale (I) dans laquelle A représente un groupement -CR6R7, R6 représentant un atome d'hydrogène et R7 représentant un groupement -OR8, R8 étant tel que défini ci-dessus. En particulier, R8 représente un groupement alkyle comprenant préférentiellement 1, 2, 3 ou 4 atomes de carbone. Encore plus préférentiellement, R8 représente un groupement alkyle substitué par un groupement aryle ou cycloalkyle, ledit groupement aryle ou cycloalkyle comportant en particulier 6 atomes de carbone.

Un autre objet particulier de l'invention concerne les composés de formule générale (I) dans laquelle X3 et X5, identiques ou différents, représentent respectivement un groupement R3 et R5, R3 et R5, identiques ou différents, représentant un groupement alkyle non substitué.

Préférentiellement, X3 et X5, identiques ou différents, représentent respectivement un groupement R3 et R5, R3 et R5, identiques ou différents, représentant un groupement alkyle non substitué, comportant préférentiellement 1, 2, 3 ou 4 atomes de carbone. Encore plus préférentiellement, X3 et X5, identiques ou différents, représentent un groupement méthyle.

Un autre aspect particulier de l'invention concerne les composés de formule générale (I) dans laquelle X4 représente un groupement G4-R4, G4 étant tel que défini ci-avant, et
R4 représentant un groupement alkyle substitué par groupementCOOR9, en particulier par COOH. Encore plus préférentiellement, X4 représente un groupement -OC(CH₃)₂COOH ou -OCH₂COOH.

Un aspect particulier de l'invention concerne les composés de formule générale (I) dans laquelle R9 représente préférentiellement un atome d'hydrogène ou un groupement alkyle comprenant 1, 2, 3, 4, 5 ou 6 atomes de carbone.

Un aspect particulier de l'invention concerne les composés de formule générale (I) dans laquelle X4 répond à la formule -OC(CH₃)₂COOR9, R9 étant tel que défini ci-avant et représentant préférentiellement un atome d'hydrogène ou un groupement alkyle comprenant 1, 2, 3, 4, 5 ou 6 atomes de carbone.

Un objet particulier de l'invention concerne les composés de formule générale (I) dans laquelle X1 représente un groupement R1 ou G1R1,
G1 étant tel que défini ci-avant, et
R1 représentant un groupement alkyle halogéné.

Préférentiellement, R1 représente un groupement alkyle halogéné comportant 1, 2 ou 3 atomes de carbone.

Encore plus préférentiellement, X1 représente un groupement -CF₃, -OCF₃, - SCF₃, -ORCF₃, R représentant un groupement alkyle tel que défini ci-dessus.

Un objet particulier de l'invention concerne les composés de formule générale (I) dans laquelle X1 représente un atome d'halogène (brome, chlore, fluor, iode). Préférentiellement, X1 représente un atome de chlore ou de brome.

Des composés faisant partie de la présente invention ainsi que des composés comparatifs sont indiqués ci-dessous:
Composé 1 : Acide 2-[2,6-diméthyl-4-[3-[4-(trifluorométhyloxy)phényl]-3-oxopropyl]phénoxy]-2-méthylpropanoïque 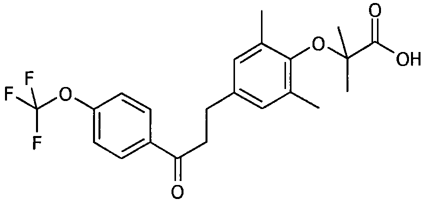
Composé 2 : Acide 2-[2,6-diméthyl-4-[3-[4-(trifluorométhylthio)phényl]-3-oxopropyl]phénoxy]-2-méthylpropanoïque 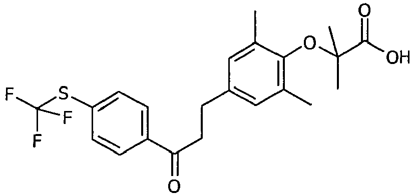
Composé 3: Acide 2-[2,6-diméthyl-4-[3-[4-bromophényl]-3-oxo-propyl]phénoxy]-2-méthylpropanoïque 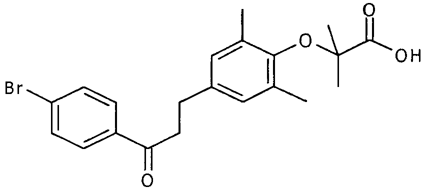
Composé 4 : Acide 2-[2,6-diméthyl-4-[3-[4-(trifluorométhyl)phényl]-3-oxopropyl]phénoxy]-2-méthylpropanoïque 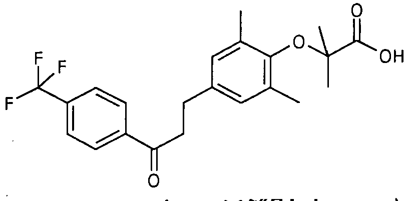
Composé 5 (comparatif): Acide 2-[4-[3-[4-chloro-2-hydroxyphényl]-3-oxo-propyl]phénylthio]-2-méthylpropanoïque 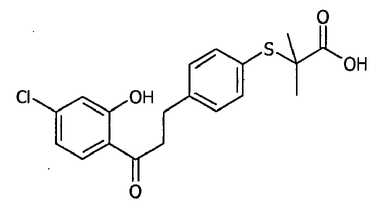
Composé 6: Acide 2-[2-méthyl-4-[3-[4-(3,3,3-trifluoropropyloxy)phényl]-3-oxopropyl]phénoxy]-2-méthylpropanoïque 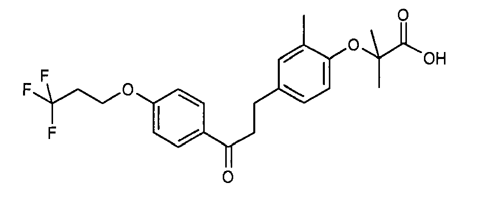
Composé 7: Acide 2-[2,6-diméthyl-4-[3-hydroxy-3-[4-(trifluorométhylthio)phényl]propyl]phénoxy]-2-méthylpropanoïque 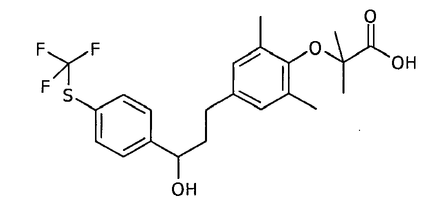
Composé 8: Acide 2-[2,6-diméthyl-4-(3-(pyridin-3-ylméthoxy)-3-(4-(trifluorométhoxy)phényl)propyl)phénoxy]-2-méthylpropanoïque 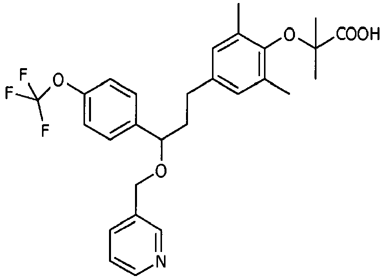
Composé 9 : Acide 2-[4-(3-(4-iodobenzyloxy)-3-(4-(trifluorométhoxy)phényl)propyl)-2,6-diméthylphénoxy]-2-méthylpropanoïque 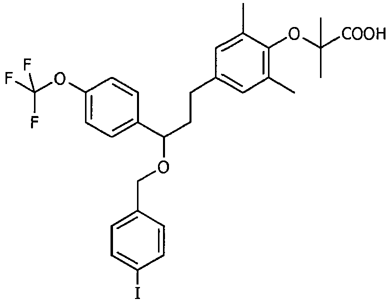
Composé 10: Acide 2-[4-(3-méthoxy-3-(4-(trifluorométhoxy)phényl)propyl)-2,6-diméthylphénoxy]-2-méthylpropanoïque 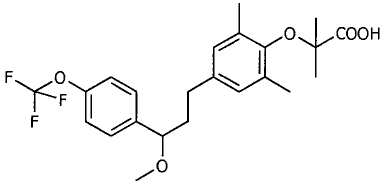
Composé 11 : Acide 2-[2,6-diméthyl-4-[3-[4-(3,3,3-trifluoropropyloxy)phényl]-3-oxopropyl]phénoxy]-2-méthylpropanoïque 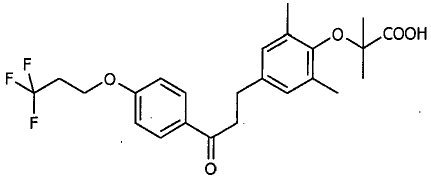
Composé 12 : Acide 2-(2,6-diméthyl-4-(3-oxo-3-(4-(2,2,2-trifluoroéthoxy)phényl)propyl)phénoxy)-2-méthylpropanoïque 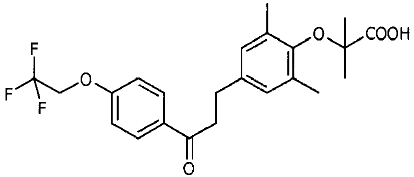
Composé 13 : Acide 2-(2,6-diméthyl-4-(3-oxo-3-(4-(2,2,2-trifluoroéthylthio)phényl)propyl)phénoxy)-2-méthylpropanoïque 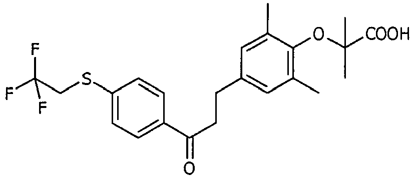
Composé 14 (comparatif): Acide 2-(4-(3-(4-chloro-2-(méthylthio)phényl)-3-oxo-propyl)-2,6-diméthylphénoxy)-2-méthylpropanoïque 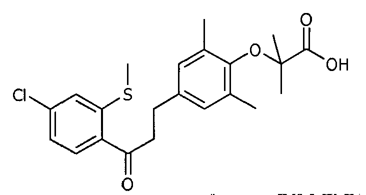
Composé 15 (comparatif): Acide 2-(4-(3-(2,4-bis(trifluorométhyl)phényl)-3-oxo-propyl)-2,6-di méthylphénoxy)-2-méthyl propa noïque 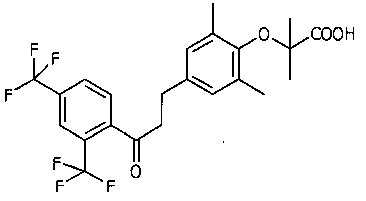
Composé 16 (comparatif): Acide 2-(4-(3-(2-fluoro-4-(trifluorométhyl)phényl)-3-oxo-propyl)-2,6-diméthylphénoxy)-2-méthylpropanoïque 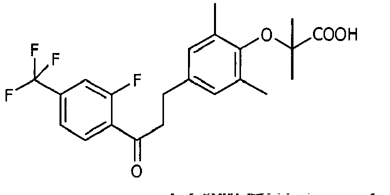
Composé 17 (comparatif): Acide 2-(4-(3-(2-fluoro-4-(2,2,2-trifluoroéthoxy)phényl)-3-oxo-propyl)-2,6-diméthylphénoxy)-2-méthylpropanoïque 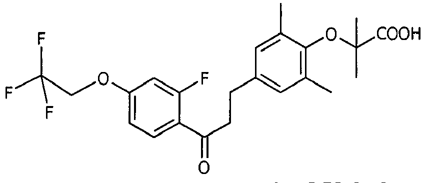
Composé 18 (comparatif): Acide 2-(2,6-diméthyl-4-(3-(2-méthyl-4-(2,2,2-trifluoroéthoxy)phényl)-3-oxo-propyl)phénoxy)-2-méthylpropanoïque 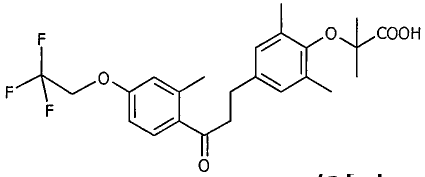
Composé 19 (comparatif): Acide 2-(4-(3-(2-méthoxy-4-(2,2,2-trifluoroéthoxy)phényl)-3-oxo-propyl)-2,6-diméthylphénoxy)-2-méthylpropanoïque 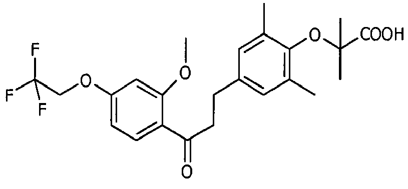
Composé 20 (comparatif): Acide 2-(4-(3-(2-hydroxy-4-(trifluorométhyl)phényl)-3-oxo-propyl)-2,6-diméthylphénoxy)-2-méthylpropanoïque 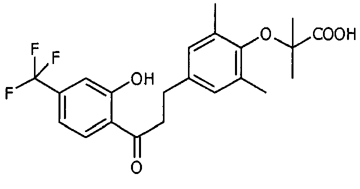
Composé 21 (comparatif): Acide 2-(4-(3-(2-méthoxy-4-(trifluorométhyl)phényl)-3-oxo-propyl)-2,6-diméthylphénoxy)-2-méthylpropanoïque 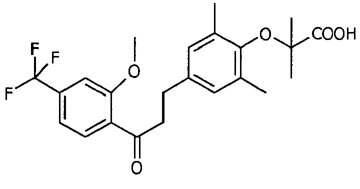
Composé 22 (comparatif): Acide 2-(2,6-diméthyl-4-(3-(2-isopropyloxy-4-(trifluorométhyl)phényl)-3-oxo-propyl)phénoxy)-2-méthylpropanoïque 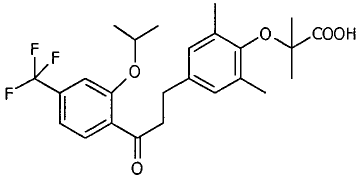
Composé 23 : Acide 2-(2,6-diméthoxy-4-(3-oxo-3-(4-(trifluorométhoxy)phényl)propyl)phénoxy)-2-méthylpropanoïque 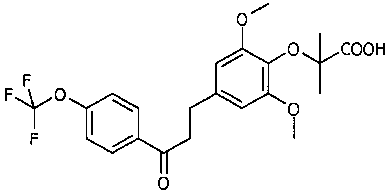
Composé 24 (comparatif): Acide 2-(4-(3-(2-fluoro-4-(trifluorométhyl)phényl)-3-oxo-propyl)-2,6-diméthoxyphénoxy)-2-méthylpropanoïque 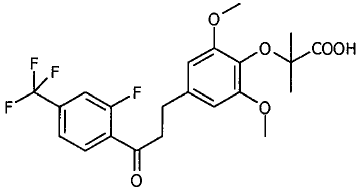
Composé 25: Acide 2-méthyl-2-(2-méthyl-4-(3-oxo-3-(4-(trifluorométhylthio)phényl)propyl)phénoxy)propanoïque 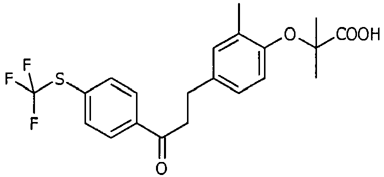
Composé 26 : Acide 2-méthyl-2-(2-méthyl-4-(3-oxo-3-(4-(trifluorométhoxy)phényl)propyl)phénoxy)propanoïque 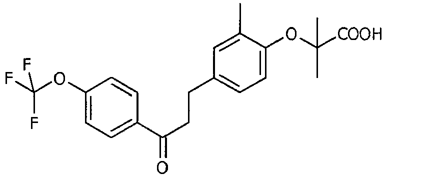
Composé 27 (comparatif): Acide 2-(4-(3-(2-fluoro-4-(trifluorométhyl)phényl)-3-oxopropyl)phénylthio)-2-méthylpropanoïque 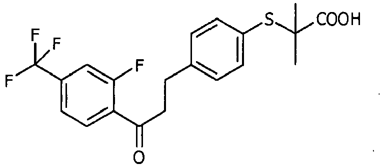
Composé 28 : Acide 2-méthyl-2-(3-(3-oxo-3-(4-(trifluorométhoxy)phényl)propyl)phénoxy)propanoïque 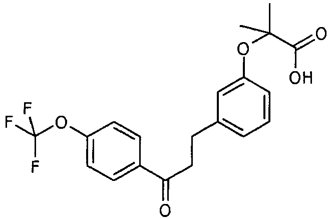
Composé 29 : Acide 2-(2,6-diméthyl-4-(3-oxo-3-(4-(trifluorométhoxy)phényl)propyl)phénoxy)propanoïque 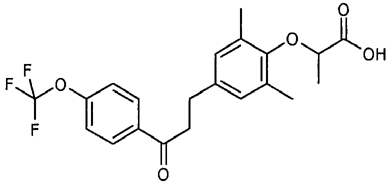
Composé 30: Acide 2-(4-(3-hydroxy-3-(4-(trifluorométhoxy)phényl)propyl)-2,6-diméthylphénoxy)-2-méthylpropanoïque 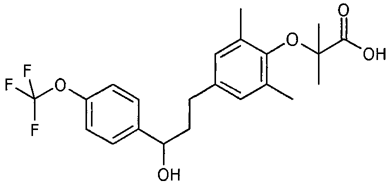
Composé 31 : 2-(2,6-diméthyl-4-(3-oxo-3-(4-(trifluorométhoxy)phényl)propyl)phénoxy)-2-méthylpropanamide 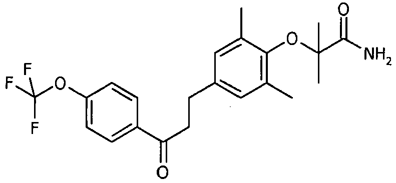
Composé 32 (comparatif): Acide 2-(4-(3-(hydroxyimino)-3-(4-(trifluorométhoxy)phényl)propyl)-2,6-diméthylphénoxy)-2-méthylpropanoïque 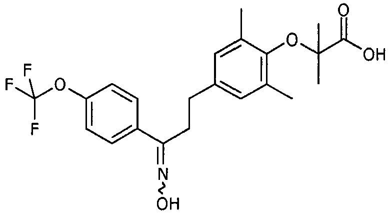
Composé 33 (comparatif): Acide 2-(4-(3-(méthoxyimino)-3-(4-(trifluorométhoxy)phényl)propyl)-2,6-diméthylphénoxy)-2-méthylpropanoïque 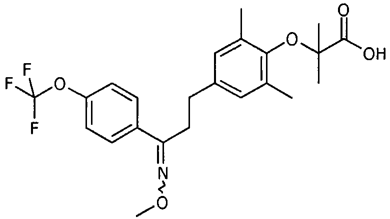
Composé 34 : Acide 4-(2,6-diméthyl-4-(3-oxo-3-(4-(trifluorométhoxy)phényl)propyl)phénoxy)-2,2-diméthylbutanoïque 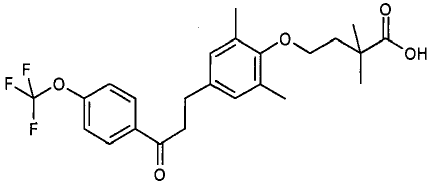
Composé 35: 2-(2,6-diméthyl-4-(3-oxo-3-(4-(trifluorométhoxy)phényl)propyl)phénoxy)-2-méthylpropanoate de tertiobutyle 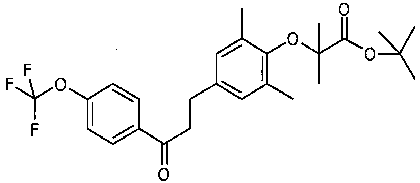
Composé 36: 2-(2,6-diméthyl-4-(3-oxo-3-(4-(trifluorométhoxy)phényl)propyl)phénoxy)-2-méthylpropanoate d'isopropyle 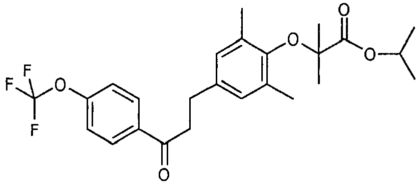
Composé 37: Acide 2,2-difluoro-2-(2,6-diméthyl-4-(3-oxo-3-(4-(trifluorométhoxy)phényl)propyl)phénoxy)acétique 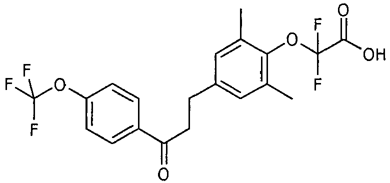
Composé 38 : Acide 2-(2-méthoxy-4-(3-oxo-3-(4-(trifluorométhoxy)phényl)propyl)phénylthio)-2-méthylpropanoïque

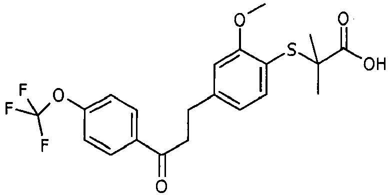

Les composés préférés de la présente invention sont ceux cités dans la revendication 7. L'invention concerne plus préférentiellement les composés suivants:
Composé 1 : Acide 2-[2,6-diméthyl-4-[3-[4-(trifluorométhoxy)phényl]-3-oxopropyl]phénoxy]-2-méthylpropanoïque ;
Composé 2 : Acide 2-[2,6-diméthyl-4-[3-[4-(trifluorométhylthio)phényl]-3-oxopropyl]phénoxy]-2-méthylpropanoïque;
Composé 3: Acide 2-[2,6-diméthyl-4-[3-[4-bromophényl]-3-oxo-propyl]phénoxy]-2-méthylpropanoïque ;
Composé 4 : Acide 2-[2,6-diméthyl-4-[3-[4-(trifluorométhyl)phényl]-3-oxopropyl]phénoxy]-2-méthylpropanoïque
Composé 11 : Acide 2-[2,6-diméthyl-4-[3-[4-(3,3,3-trifluoropropyloxy)phényl]-3-oxopropyl]phénoxy]-2-méthylpropanoïque
Composé 36 : 2-(2,6-diméthyl-4-(3-oxo-3-(4-(trifluorométhoxy)phényl)propyl)phénoxy)-2-méthylpropanoate d'isopropyle

Les composés de la présente invention comprennent leurs stéréoisomères (diastéréoisomères, énantiomères), purs ou en mélange, leurs mélanges racémiques, leurs isomères géométriques, leurs tautomères, leurs sels, leurs hydrates, leurs solvates, leurs formes solides ainsi que leurs mélanges.

Les composés selon l'invention peuvent contenir un ou plusieurs centres asymétriques. La présente invention inclut les stéréoisomères (diastéréoisomères, énantiomères), purs ou en mélange, ainsi que les mélanges racémiques et les isomères géométriques. Quand un mélange énantiomériquement pur (ou enrichi) est souhaité, il pourra être obtenu soit par purification du produit final ou d'intermédiaires chiraux, soit par synthèse asymétrique suivant des méthodes connues de l'homme de métier (utilisant par exemple des réactifs et catalyseurs chiraux). Certains composés selon l'invention peuvent avoir différentes formes tautomères stables et toutes ces formes ainsi que leurs mélanges sont inclus dans l'invention.

La présente invention concerne également les sels « pharmaceutiquement acceptables » des composés selon l'invention. D'une manière générale, ce terme désigne les sels peu ou non toxiques obtenus à partir de bases ou d'acides, organiques ou inorganiques. Ces sels peuvent être obtenus lors de l'étape de purification finale du composé selon l'invention ou par incorporation du sel sur le composé déjà purifié.

Certains composés selon l'invention et leurs sels pourraient être stables sous plusieurs formes solides. La présente invention inclut toutes les formes solides des composés selon l'invention ce qui inclut les formes amorphes, polymorphes, mono- et poly-cristallines.

Les composés selon l'invention peuvent exister sous forme libre ou sous forme solvatée, par exemple avec des solvants pharmaceutiquement acceptables tels que l'eau (hydrates) ou l'éthanol.

Les composés selon l'invention marqués par un ou des isotopes sont également inclus dans l'invention : ces composés sont structurellement identiques mais diffèrent par le fait qu'au moins un atome de la structure est remplacé par un isotope (radioactif ou non). Des exemples d'isotopes pouvant être inclus dans la structure des composés selon l'invention peuvent être choisis parmi l'hydrogène, le carbone, l'oxygène, le soufre tels que ²H, ³H, ¹³C, ¹⁴C, ¹⁸O, ¹⁷O, ³⁵S respectivement. Les isotopes radioactifs ³H et ¹⁴C sont particulièrement préférés car faciles à préparer et à détecter dans le cadre d'études de biodisponibilité *in vivo* des substances. Les isotopes lourds (tels que ²H) sont particulièrement préférés car ils sont utilisés comme standards internes dans des études analytiques.

La présente invention a également pour objet un procédé de synthèse des composés de formule générale (I) telle que définie ci-avant.

Le procédé de la présente invention comprend :
- une étape de mise en contact (i) en milieu basique ou en milieu acide d'au moins un composé de formule (A) avec au moins un composé de formule (B): 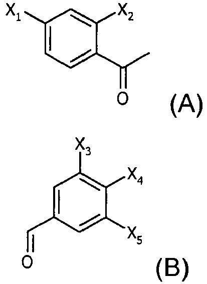 dans lesquelles X1, X2, X3, X4 et X5 ont les définitions données précédemment, puis,
- puis (ii) un étape de réduction des composés ainsi obtenus,
- et éventuellement (iii) une étape d'insertion de groupements fonctionnels.
Les conditions de mise en oeuvre de l'étape (i) en milieu acide ou basique et de l'étape (ii) sont à la portée de l'homme du métier et peuvent varier dans une large mesure. Les protocoles de synthèse peuvent être en particulier ceux présentés dans la partie « exemples » de la présente invention.

La mise en contact de ces deux composés est avantageusement réalisée de manière stoechiométrique. Elle est réalisée de préférence à une température ambiante (entre environ 18°C et 25°C) et à pression atmosphérique.

En milieu basique, la réaction est de préférence réalisée en présence d'une base forte, tel qu'un hydroxyde de métal alcalin, comme l'hydroxyde de sodium ou un alcoolate de métal alcalin comme l'éthylate de sodium.

En milieu acide, la réaction est de préférence réalisée en présence d'un acide fort, tel que l'acide chlorhydrique.

Les composés ainsi obtenus peuvent être isolés par des méthodes classiques et connues de l'homme du métier. Ils peuvent être ensuite utilisés notamment à titre de médicaments ou de produits cosmétiques.

La présente invention a aussi pour objet les composés tels que décrits ci-avant, à titre de médicaments.

La présente invention a également pour objet une composition pharmaceutique comprenant, dans un support acceptable sur le plan pharmaceutique, au moins un composé tel que décrit ci-dessus, éventuellement en association avec un ou plusieurs autres principes actifs thérapeutiques et/ou cosmétiques.

Il s'agit avantageusement d'une composition pharmaceutique pour le traitement des complications associées au syndrome métabolique, de l'insulino-résistance, du diabète, des dyslipidémies, de l'athérosclérose, des maladies cardiovasculaires, de l'obésité, de l'hypertension, des maladies inflammatoires (asthme, etc.), des pathologies neurodégénératives (Alzheimer, etc.), ou des cancers, etc. La composition pharmaceutique selon l'invention est préférentiellement utilisée pour traiter les dyslipidémies.

Il s'agit préférentiellement d'une composition pharmaceutique pour traiter les facteurs de risque cardiovasculaire liés aux dérèglements du métabolisme lipidique et/ou glucidique (hyperlipidémie, diabète de type II, obésité etc.) en permettant la diminution du risque global.

Un autre objet de l'invention concerne une composition nutritionnelle comprenant au moins un composé tel que décrit ci-dessus.

Un autre objet de l'invention réside dans l'utilisation d'au moins un composé tel que décrit ci-avant pour la préparation de compositions pharmaceutiques destinées au traitement de diverses pathologies, notamment liées à des troubles du métabolisme parmi lesquelles on peut citer les dyslipidémies. Plus généralement, l'invention a pour objet l'utilisation d'au moins un composé tel que décrit ci-avant pour la préparation de compositions pharmaceutiques destinées à traiter les facteurs de risques pour les maladies cardiovasculaires liés aux dérèglements du métabolisme des lipides et/ou des glucides et destinées à diminuer ainsi le risque global.

A titre d'exemple (et de manière non limitative), les composés selon l'invention pourront de manière avantageuse être administrés en combinaison avec d'autres agents thérapeutiques et/ou cosmétiques, commercialisés ou en développement, tels que :
- des anti-diabétiques : les insulinosécréteurs (sulfonylurées (glibenclamide, glimépiride, gliclazide, etc.) et glinides (répaglinide, natéglinide, etc.)), les inhibiteurs de l'alpha-glucosidase, les agonistes PPARγ (thiazolidinediones telles que rosiglitazone, pioglitazone), les agonistes mixtes PPARα/PPARγ (tesaglitazar, muraglitazar), les pan-PPAR (composés activant simultanément les 3 isoformes PPAR), des biguanides (metformine), les inhibiteurs de la Dipeptidyl Peptidase IV (MK-431, vildagliptin), les agonistes du Glucagon-Like Peptide-1 (GLP-1) (exenatide), etc.
- l'insuline
- des molécules hypolipémiantes et/ou hypocholestérolémiantes : les fibrates (fenofibrate, gemfibrozil), les inhibiteurs de la HMG CoA réductase ou hydroxylméthylglutaryl Coenzyme A reductase (les statines telles que atorvastatine, simvastatine, fluvastatine), les inhibiteurs de l'absorption du cholestérol (ezetimibe, phytostérols), les inhibiteurs de la CETP ou Cholesteryl Ester Transfer Protein (torcetrapib), les inhibiteurs de l'ACAT ou Acyl-Coenzyme A cholesterol acylTransferase (Avasimibe, Eflucimibe), les inhibiteurs MTP (Microsomal Triglyceride Transfer Protein), les agents séquestrants des acides biliaires (cholestyramine), la vitamine E, les acides gras poly-insaturés, les acides gras oméga 3, les dérivés de type acide nicotinique (niacine), etc.
- des agents anti-hypertenseurs et les agents hypotenseurs : les inhibiteurs ACE (Angiotensin-Converting Enzyme) (captopril, enalapril, ramipril ou quinapril), les antagonistes du récepteur de l'angiotensine II (losartan, valsartan, telmisartan, eposartan, irbesartan, etc.), les béta-bloquants (atenolol, metoprolol, labetalol, propranolol), les diurétiques thiazidiques et non thiazidiques (furosemide, indapamide, hydrochlorthiazide, anti-aldosterone), les vasodilatateurs, les bloquants des canaux calciques (nifedipine, felodipine ou amlodipine, diltiazem ou verapamil), etc.
- des agents anti-plaquettaires : Aspirine, Ticlopidine, Dipyridamol, Clopidogrel, flurbiprofen, etc.
- des agents anti-obésité : Sibutramine, les inhibiteurs de lipases (orlistat), les agonistes et antagonistes PPARδ, les antagonistes du récepteur cannabinoïde CB1 (rimonabant), etc.
- des agents anti-inflammatoires : par exemple, les corticoïdes (prednisone, betamethasone, dexamethasone, prednisolone, méthylprednisolone, hydrocortisone, etc.), les AINS ou Anti-Inflammatoires Non Stéroidiens dérivés de l'indole (indomethacine, sulindac), les AINS du groupe des arylcarboxyliques (acide tiaprofenique, diclofenac, etodolac, flurbiprofen, ibuprofen, ketoprofen, naproxen, nabumetone, alminoprofen), les AINS dérivés de l'oxicam (meloxicam, piroxicam, tenoxicam), les AINS du groupe des fénamates, les inhibiteurs sélectifs de la COX2 (celecoxib, rofecoxib), etc.
- des agents anti-oxydants : par exemple le probucol, etc.
- des agents utilisés dans le traitement de l'insuffisance cardiaque : les diurétiques thiazidiques ou non thiazidiques (furosemide, indapamide, hydrochlorthiazide, anti-aldosterone), les inhibiteurs de l'ACE (captopril, enalapril, ramipril ou quinapril), les digitaliques (digoxin, digitoxin), les béta bloquants (atenolol, metoprolol, labetalol, propranolol), les inhibiteurs de Phosphodiesterases (enoximone, milrinone), etc.
- des agents utilisés pour le traitement de l'insuffisance coronaire : les béta-bloquants (atenolol, metoprolol, labetalol, propranolol), les bloquants des canaux calciques (nifedipine, felodipine ou amlodipine, bepridil, diltiazem ou verapamil), les agents donneurs de NO (trinitrine, isosorbide dinitrate, molsidomine), l'Amiodarone, etc.
- des anticancéreux : les agents cytotoxiques (agents intéragissants avec l'ADN, agents alkylants, cisplatine et dérivés), les agents cytostatiques (les analogues GnRH (Gonatropin-Releasing Hormone), les analogues de la somatostatine, les progestatifs, les anti-oestrogènes, les inhibiteurs de l'aromatase, etc.), les modulateurs de la réponse immunitaire (interférons, IL2, etc.), etc.
- des anti-asthmatiques tels que des bronchodilatateurs (agonistes des récepteurs béta 2), des corticoïdes, le cromoglycate, les antagonistes du récepteur aux leucotriènes (montelukast), etc.
- des corticoïdes utilisés dans le traitement des pathologies de la peau telles que le psoriasis et les dermatites
- des vasodilatateurs et/ou des agents anti-ischémiques (buflomedil, extrait de Ginkgo Biloba, naftidrofuryl, pentoxifylline, piribédil), etc.

L'invention concerne également une méthode de traitement des pathologies liées au métabolisme des lipides et/ou des glucides comprenant l'administration à un sujet, notamment humain, d'une quantité efficace d'un composé ou d'une composition pharmaceutique tels que définis ci-avant. Au sens de l'invention le terme «une quantité efficace » se réfère à une quantité du composé suffisante pour produire le résultat biologique désiré. Au sens de la présente invention le terme « sujet » signifie un mammifère et plus particulièrement un humain.

Le terme « traitement » désigne le traitement curatif, symptomatique ou préventif. Les composés de la présente invention peuvent ainsi être utilisés chez des sujets (comme les mammifères, en particulier humains) atteints d'une maladie déclarée. Les composés de la présente invention peuvent aussi être utilisés pour retarder ou ralentir la progression ou prévenir une progression plus en avant de la maladie, améliorant ainsi la condition des sujets. Les composés de la présente invention peuvent enfin être administrés aux sujets non malades, mais qui pourraient développer normalement la maladie ou qui ont un risque important de développer la maladie.

Les compositions pharmaceutiques selon l'invention comprennent avantageusement un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, le polysorbate 80, le mannitol, la gélatine, le lactose, des huiles végétales, l'acacia, les liposomes, etc. Les compositions peuvent être formulées sous forme de suspensions injectables, gels, huiles, comprimés, suppositoires, poudres, gélules, capsules, aérosols, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

Les composés ou compositions selon l'invention peuvent être administrés de différentes manières et sous différentes formes. Ainsi, ils peuvent être par exemple administrés de manière systémique, par voie orale, parentérale, par inhalation ou par injection, comme par exemple par voie intraveineuse, intra-musculaire, sous-cutanée, trans-dermique, intra-artérielle, etc. Pour les injections, les composés sont généralement conditionnés sous forme de suspensions liquides, qui peuvent être injectées au moyen de seringues ou de perfusions, par exemple.

Il est entendu que le débit et/ou la dose injectée peuvent être adaptés par l'homme du métier en fonction du patient, de la pathologie, du mode d'administration, etc. Typiquement, les composés sont administrés à des doses pouvant varier entre 1 µg et 2 g par administration, préférentiellement de 0,1 mg à 1 g par administration. Les administrations peuvent être quotidiennes voire répétées plusieurs fois par jour, le cas échéant. D'autre part, les compositions selon l'invention peuvent comprendre, en outre, d'autres agents ou principes actifs.

### LEGENDES DES FIGURES

### Abréviations employées sur les figures:

- Cpd = composé
- Ctrl = contrôle
- mpk = mg/kg/jour
- LDL-cholesterol = Low Density Lipoprotein cholesterol
- HDL-cholesterol = High Density Lipoprotein cholesterol
- VLDL-cholesterol = Very Low Density Lipoprotein cholesterol

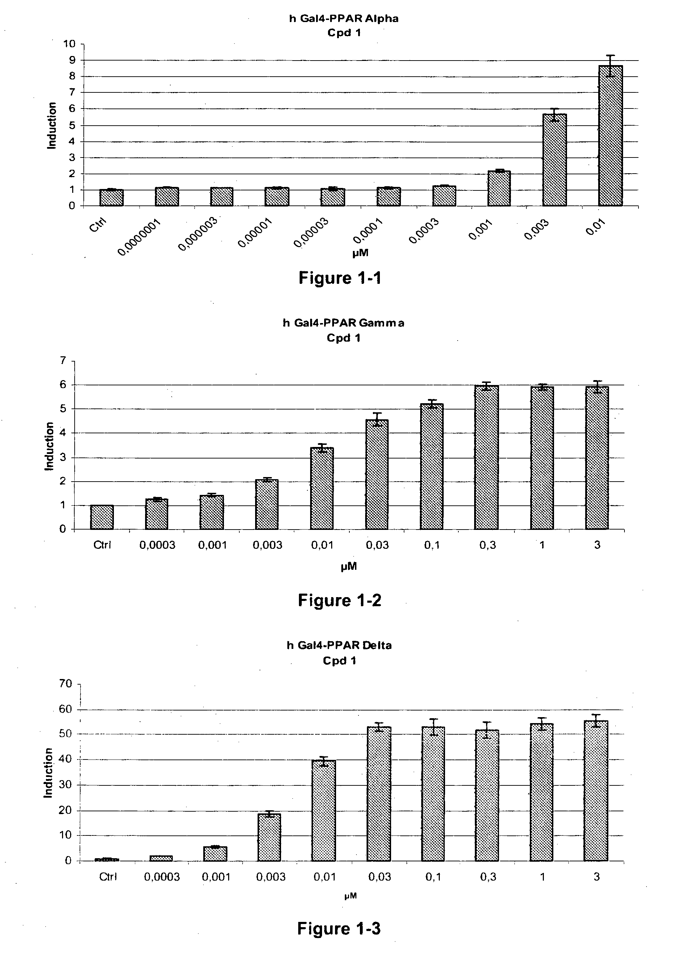

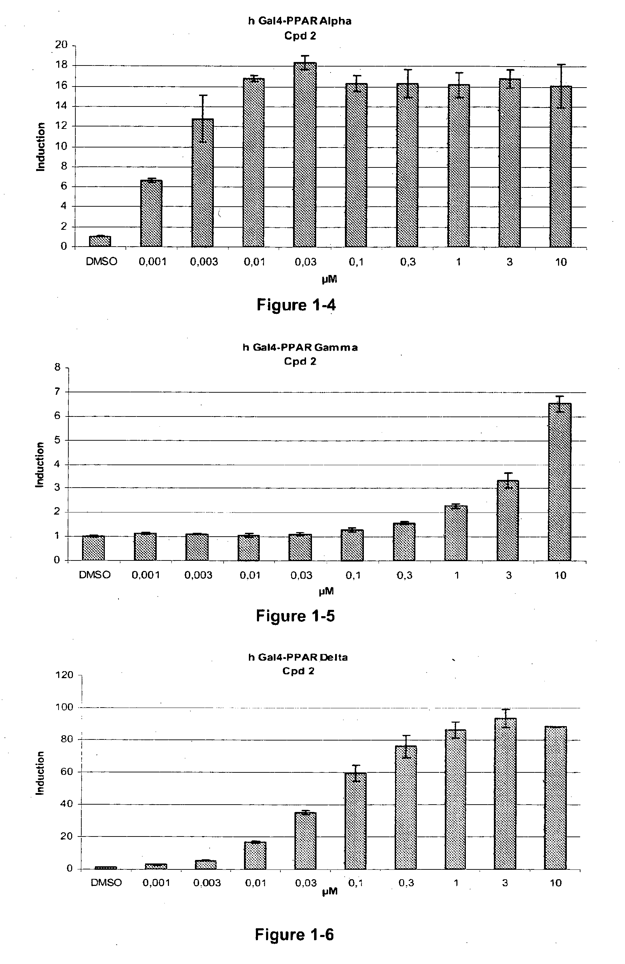

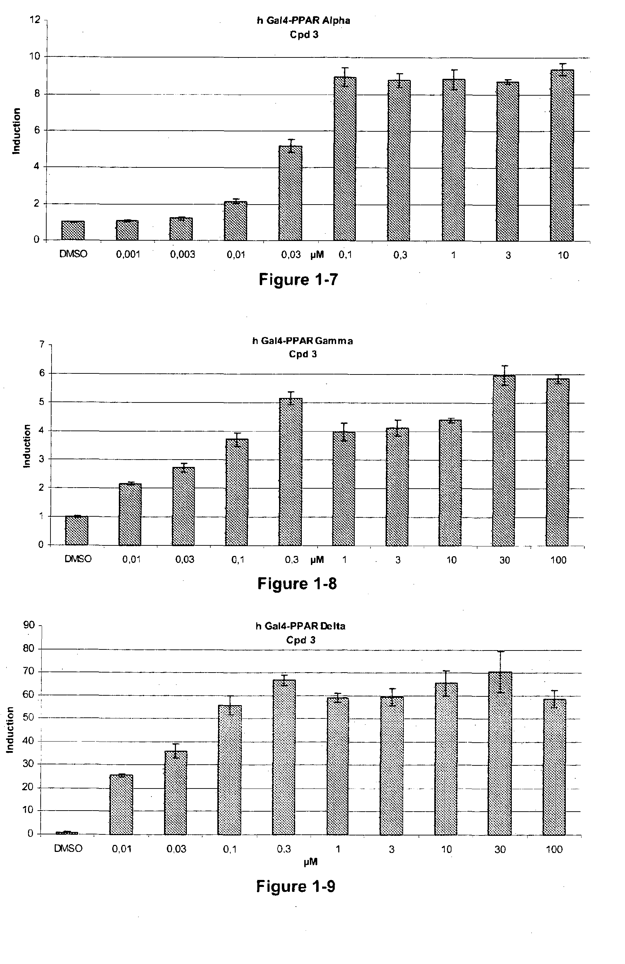

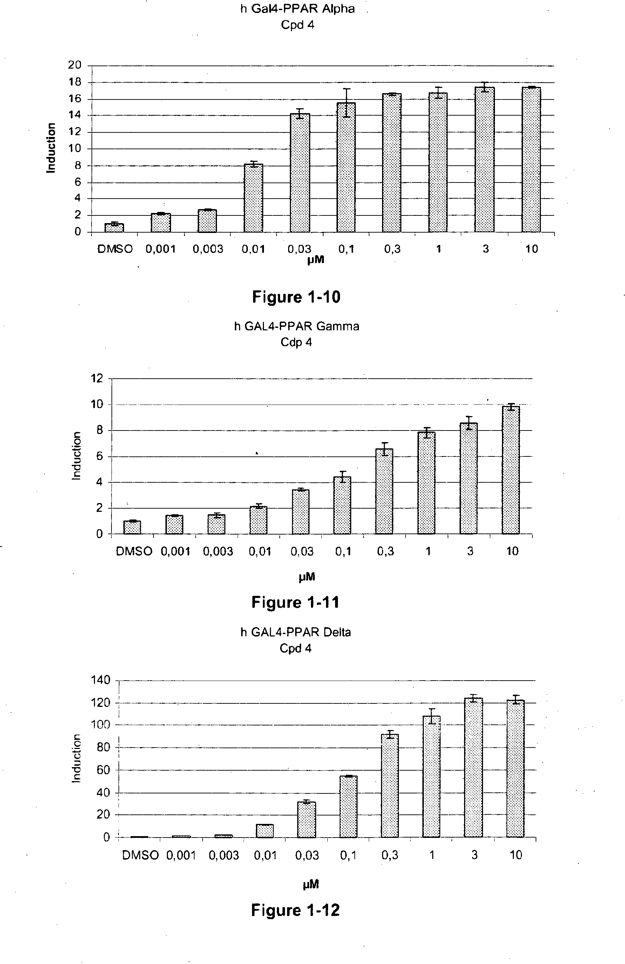

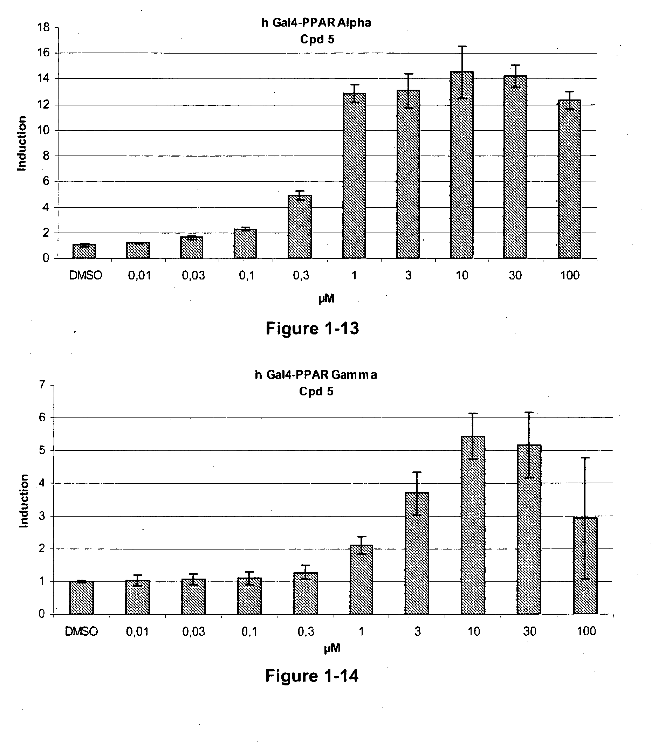

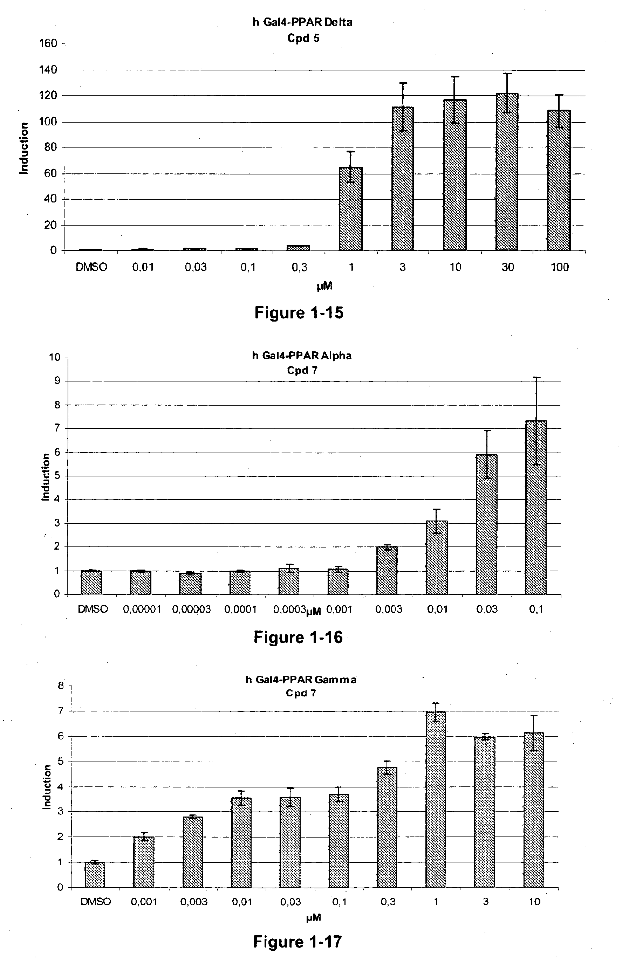

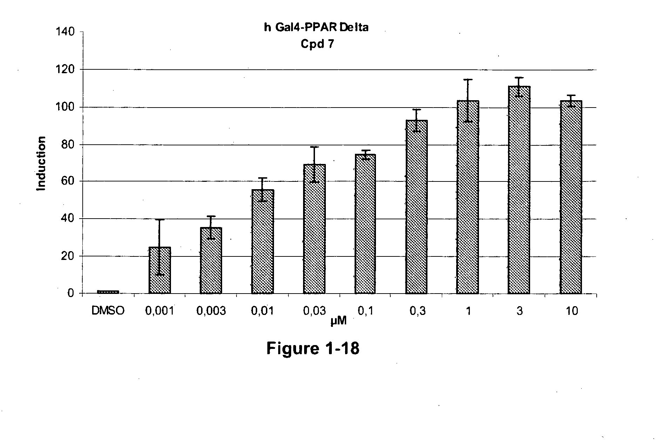

### Figures 1-1 à 1-18: Evaluation in vitro des propriétés activatrices des PPARs des composés selon l'invention en fonction de la dose

L'activation des PPAR est évaluée *in vitro* sur une lignée de fibroblastes de rein de singe (COS-7), par la mesure de l'activité transcriptionnelle de chimères constituées du domaine de liaison à l'ADN du facteur de transcription Gal4 de la levure et du domaine de liaison au ligand des différents PPAR.

Les composés sont testés à des doses comprises entre 10⁻⁷ et 100 µM sur les chimères Gal4-PPAR α, γ, δ. Le facteur d'induction, c'est-à-dire le rapport entre la luminescence induite par le composé et la luminescence induite par le contrôle, est mesuré pour chaque condition. Plus le facteur d'induction est élevé, plus le composé a un caractère activateur de PPAR.
- Figures 1-1, 1-2, 1-3: Evaluation *in vitro* des propriétés activatrices de PPARα, γ, δ du composé 1
- Figures 1-4, 1-5, 1-6: Evaluation *in vitro* des propriétés activatrices de PPARα, γ, δ du composé 2
- Figures 1-7, 1-8, 1-9: Evaluation *in vitro* des propriétés activatrices de PPARα, γ, δ du composé 3
- Figures 1-10, 1-11, 1-12: Evaluation *in vitro* des propriétés activatrices de PPARα, γ, δ du composé 4
- Figures 1-13, 1-14, 1-15: Evaluation *in vitro* des propriétés activatrices de PPARα, γ, δ du composé 5
- Figures 1-16, 1-17, 1-18: Evaluation *in vitro* des propriétés activatrices de PPARα, γ, δ du composé 7

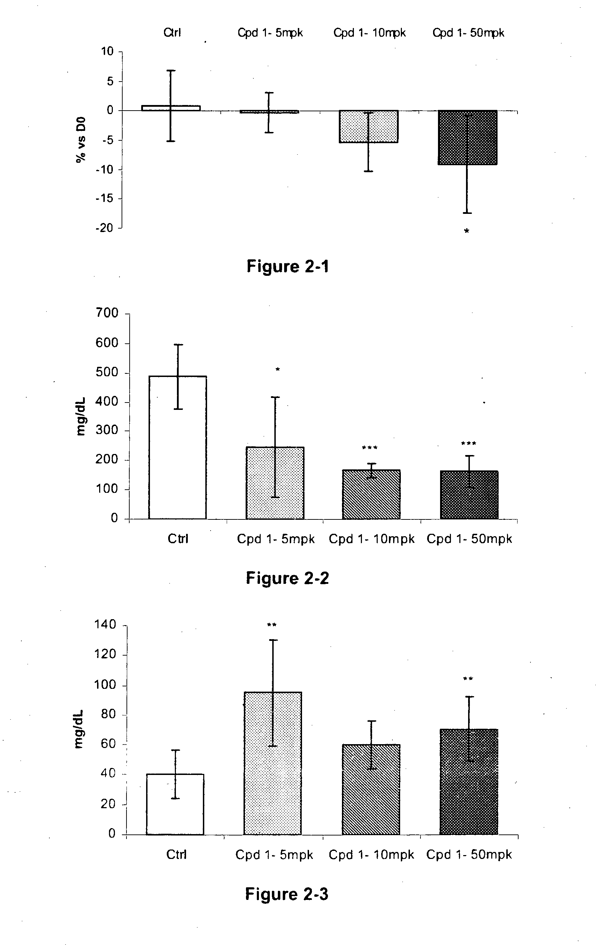

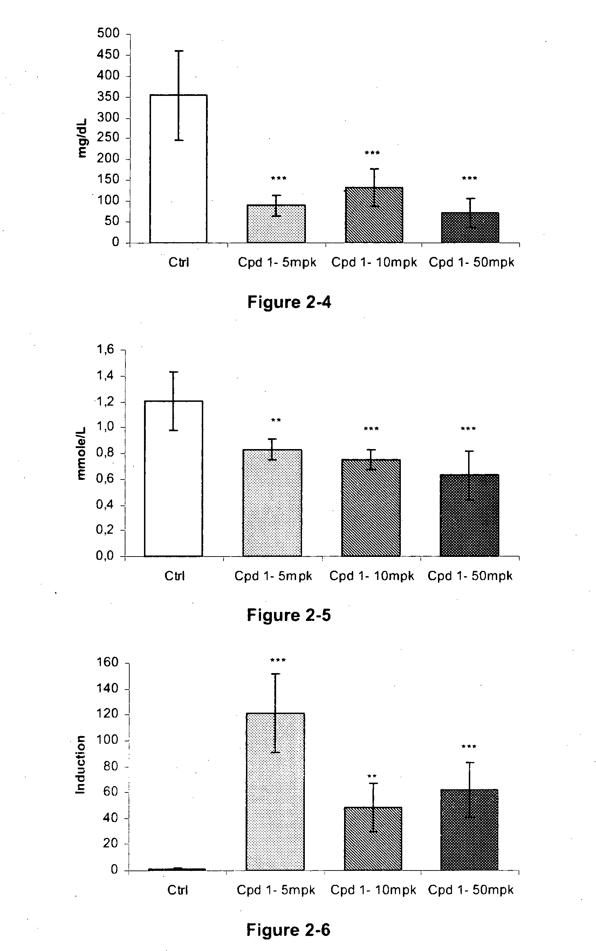

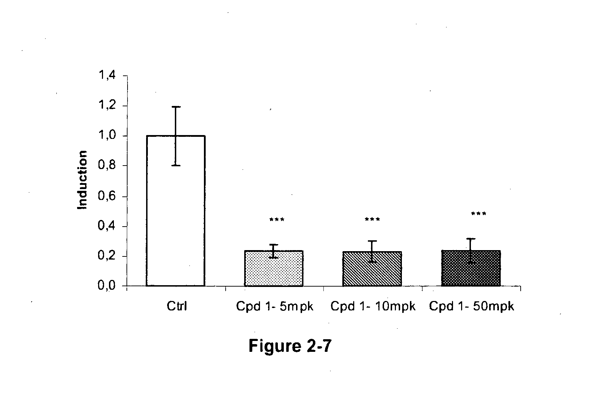

### Figures 2-1 à 2-7: Evaluation in vivo, chez la souris ApoE2/E2, des propriétés sur le poids corporel, des propriétés hypolipémiantes et des propriétés stimulatrices de la synthèse de HDL-cholestérol des composés selon l'invention

L'effet des composés selon l'invention est évalué *in vivo* chez la souris humanisée pour l'isoforme E2 de l'apolipoproteine E (E2/E2).

Le poids corporel, les taux de cholestérol total, de HDL-cholestérol, de triglycérides et d'acides gras libres plasmatiques sont mesurés chez la souris dyslipidémique E2/E2 après 8 jours de traitement par voie orale avec les composés selon l'invention. Ces paramètres sont comparés à ceux obtenus avec des animaux contrôles (non traités par les composés selon l'invention) : la différence mesurée témoigne de l'effet sur le poids corporel et de l'effet hypolipémiant des composés selon l'invention
- Figure 2-1 : Gain de poids corporel après 8 jours de traitement avec le composé 1, administré à 5, 10 et 50 mpk
- Figiure 2-2 : Taux de cholestérol plasmatique après 8 jours de traitement avec le composé 1, administré à 5, 10 et 50 mpk ;
- Figure 2-3: Taux de HDL-cholestérol plasmatique après 8 jours de traitement avec le composé 1, administré à 5, 10 et 50 mpk
- Figure 2-4 : Taux de triglycérides plasmatiques après 8 jours de traitement avec le composé 1, administré à 5, 10 et 50 mpk .
- Figiure 2-5 : Taux d'acides gras libres plasmatiques après 8 jours de traitement avec le composé 1, administré à 5, 10 et 50 mpk.

L'efficacité des composés selon l'invention est aussi évaluée par mesure, dans le tissu hépatique, de l'expression de gènes impliqués dans le métabolisme lipidique et/ou glucidique et la dissipation d'énergie. Les niveaux d'expression de chaque gène sont normalisés par rapport au niveau d'expression du gène de référence 36B4. Le facteur d'induction, c'est-à-dire le rapport entre le signal relatif (induit par le composé selon l'invention) et la moyenne des valeurs relatives du groupe contrôle, est ensuite calculé. Plus ce facteur est élevé, plus le composé a un caractère activateur d'expression génique. Le résultat final est représenté comme moyenne des valeurs d'induction dans chaque groupe expérimental.
- Figure 2-6: Expression de PDK4 (Pyruvate Deshydrogenase Kinase, isoforme 4) dans le tissu hépatique, chez la souris E2/E2, après 8 jours de traitement avec le composé 1, administré à 5, 10 et 50 mpk
- Figure 2-7: Expression de l'ApoCIII (Apolipoprotéine C3) dans le tissu hépatique, chez la souris E2/E2, après 8 jours de traitement avec le composé 1, administré à 5, 10 et 50 mpk

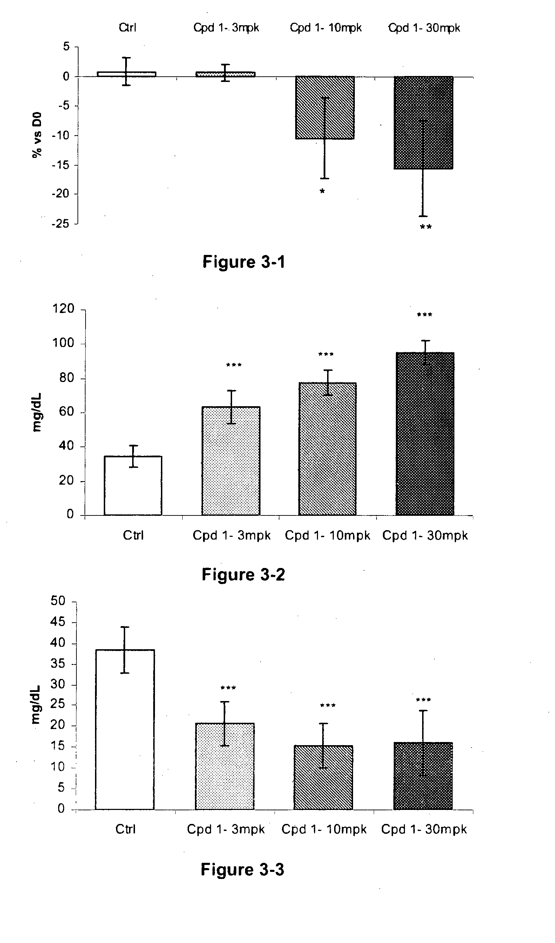

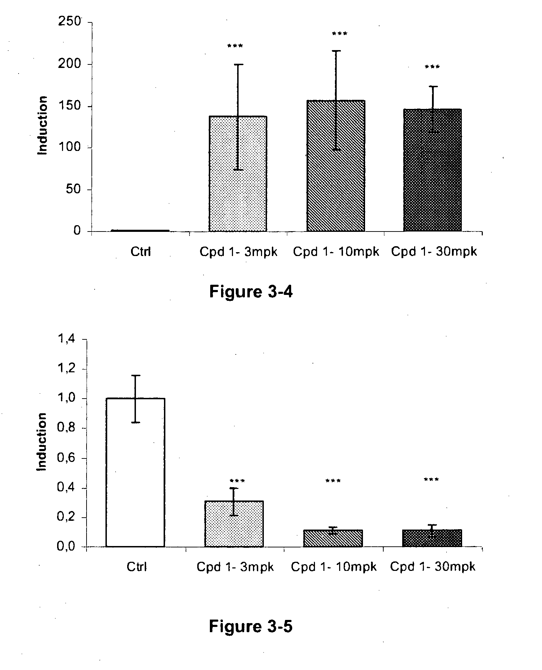

### Figures 3-1 à 3-5: Evaluation in vivo, chez la souris C57BI6, des propriétés sur le poids corporel, des propriétés hypolipémiantes et stimulatrices de la synthèse de HDL-cholestérol des composés selon l'invention.

L'effet des composés selon l'invention est évalué *in vivo* chez la souris C57BI6 par la mesure de l'évolution du poids corporel, la mesure des taux de HDL-cholestérol et de triglycérides plasmatiques après 14 jours de traitement par voie orale avec les composés selon l'invention. Ces paramètres sont comparés à ceux obtenus avec des animaux contrôles (non traités par les composés selon l'invention) : la différence mesurée témoigne de l'effet sur le poids corporel et de l'effet hypolipémiant des composés selon l'invention.
- Figure 3-1 : Gain de poids corporel après 14 jours de traitement avec le composé 1, administré à 3, 10 et 30 mpk
- Figure 3-2: Taux de HDL-cholestérol plasmatique après 14 jours de traitement avec le composé 1, administré à 3, 10 et 30 mpk
- Figure 3-3: Taux de triglycérides plasmatiques après 14 jours de traitement avec le composé 1, administré à 3, 10 et 30 mpk

L'efficacité des composés selon l'invention est aussi évaluée par mesure, dans le tissu hépatique de l'expression de gènes impliqués dans le métabolisme lipidique. Les niveaux d'expression de chaque gène sont normalisés par rapport au niveau d'expression du gène de référence 36B4. Le facteur d'induction, est ensuite calculé. Plus ce facteur est élevé, plus le composé a un caractère activateur d'expression génique. Le résultat final est représenté comme moyenne des valeurs d'induction dans chaque groupe expérimental.
- Figure 3-4: Expression de PDK4 (Pyruvate Deshydrogenase Kinase, isoforme 4) dans le tissu hépatique, chez la souris C57BI6, après 14 jours de traitement avec le composé 1, administré à 3, 10 et 30 mpk
- Figure 3-5: Expression de l'ApoClll (Apolipoprotéine C3) dans le tissu hépatique, chez la souris C57BI6, après 14 jours de traitement avec le composé 1, administré à 3, 10 et 30 mpk

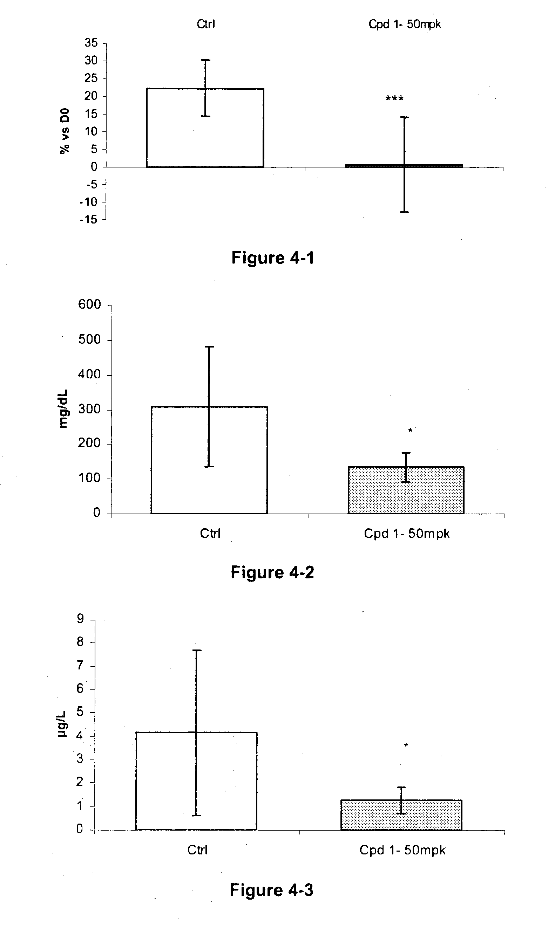

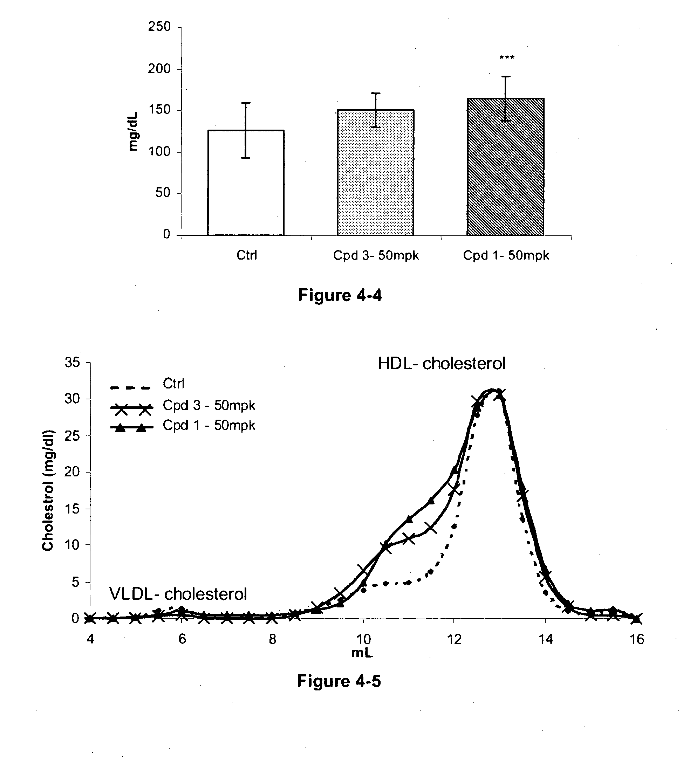

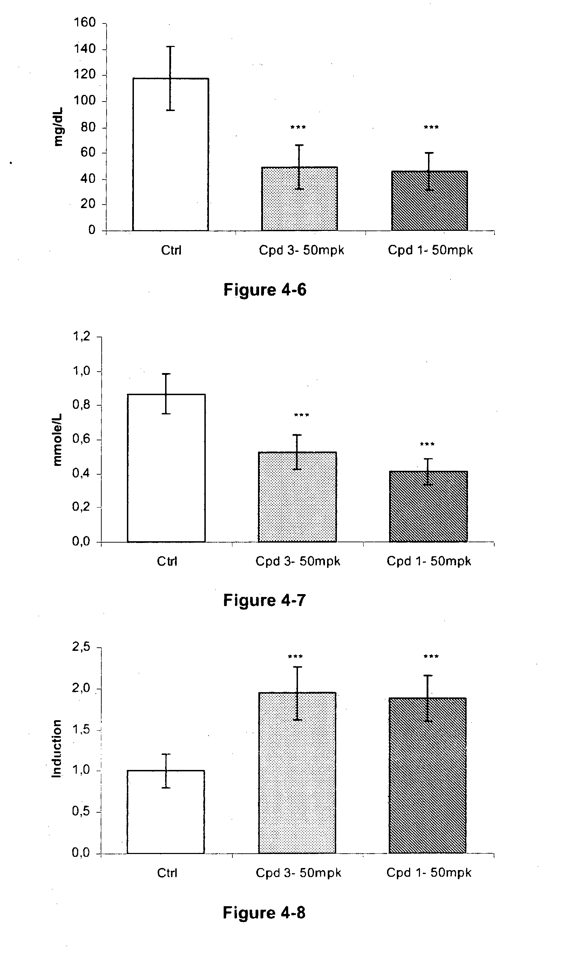

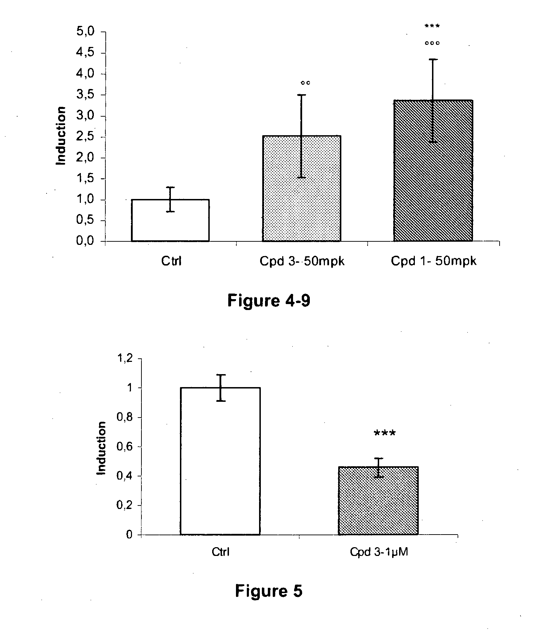

### Figures 4-1 à 4-9: Evaluation in vivo, chez la souris db/db, des propriétés sur le poids corporel, des propriétés antidiabétiques, hypolipémiantes et stimulatrices de la synthèse de HDL-cholestérol des composés selon l'invention

L'effet des composés selon l'invention est évalué *in vivo* chez la souris db/db par la mesure de l'évolution du poids corporel, la mesure des taux de glucose, d'insuline, de cholestérol total et de triglycérides plasmatiques ainsi que par l'analyse de la répartition du cholestérol dans les différentes fractions lipoprotéiques plasmatiques après 28 jours de traitement par voie orale avec les composés selon l'invention. Ces paramètres sont comparés à ceux obtenus avec des animaux contrôles (non traités par les composés selon l'invention) : la différence mesurée témoigne de l'effet sur le poids corporel, sur l'insulino-résistance, l'effet hypolipémiant des composés selon l'invention.
- Figure 4-1 : Gain de poids corporel après 28 jours de traitement avec le composé 1, administré à 50 mpk
- Figure 4-2 : Glycémie après 28 jours de traitement avec le composé 1, administré à 50 mpk
- Figure 4-3 : Insulinémie après 28 jours de traitement avec le composé 1, administré à 50 mpk
- Figure 4-4 : Taux de cholestérol plasmatique après 14 jours de traitement avec le composé 1 et le composé 3, administrés à 50 mpk
- Figure 4-5: Répartition du cholestérol dans les différentes fractions lipoprotéiques plasmatiques après 28 jours de traitement avec le composé 1 et le composé 3, administrés à 50 mpk
- Figure 4-6 : Taux de triglycérides plasmatiques après 28 jours de traitement avec le composé 1 et le composé 3, administrés à 50 mpk
- Fiqure 4-7 : Taux d'acides gras libres plasmatiques après 28 jours de traitement avec le composé 1 et le composé 3, administrés à 50 mpk.

L'efficacité des composés selon l'invention est aussi évaluée par mesure, dans les tissus hépatiques et musculaires (squelettiques), de l'expression de gènes impliqués dans le métabolisme glucidique et la dissipation d'énergie. Les niveaux d'expression de chaque gène sont normalisés par rapport au niveau d'expression des gènes de référence 36B4 dans le tissu hépatique ou 18S dans le muscle squelettique gastrocnémien. Le facteur d'induction, c'est-à-dire le rapport entre le signal relatif (induit par le composé selon l'invention) et la moyenne des valeurs relatives du groupe contrôle, est ensuite calculé. Plus ce facteur est élevé, plus le composé a un caractère activateur d'expression génique. Le résultat final est représenté comme moyenne des valeurs d'induction dans chaque groupe expérimental.
- Figure 4-8: Expression de PDK4 (Pyruvate Deshydrogenase Kinase, isoforme 4) dans le tissu hépatique, chez la souris db/db, après 28 jours de traitement avec le composé 1 et le composé 3, administrés à 50 mpk
- Figure 4-9: Expression d'UCP2 (uncoupling protein 2) dans le muscle squelettique, chez la souris db/db, après 28 jours de traitement avec les composés 1 et 3, administrés à 50 mpk

### Figure 5 : Evaluation in vitro des propriétés anti-inflammatoires des composés selon l'invention par mesure de la sécrétion de MCP1 par des monocytes traités avec les composés selon l'invention et stimulés avec du PMA

Les effets anti-inflammatoires des composés selon l'invention ont été évalués par la mesure de la sécrétion de MCP1 (Monocyte Chemotactic Protein-1) par des monocytes THP1 traités pendant 24 heures avec les composés selon l'invention et stimulés simultanément avec du PMA (Phorbol 12-Myristate 13-Acetate, provoque une réponse inflammatoire des cellules et leur différentiation en macrophages). Plus la quantité de MCP-1 sécrétée est diminuée, plus le composé selon l'invention inhibe la réaction inflammatoire.

### ANALYSES STATISTIQUES

Les études statistiques réalisées consistent en un test T de student (°/°°/°°°) et/ou une Analyse de la Variance univariée à un facteur (ANOVA), suivie d'un test de Tukey (*/**/***). Les résultats sont comparés par rapport au groupe contrôle selon la valeur du paramètre p :
°/* : p<0,05; °°/** : p<0,01 ; °°°/*** : p<0,001.

### EXEMPLES

Les réactifs et catalyseurs usuels sont disponibles commercialement (Aldrich, Alfa Aesar, Acros, Fluka ou Lancaster selon les cas).

Les spectres de Résonance Magnétique Nucléaire du Proton (RMN ¹H) ont été enregistrés sur un spectromètre Bruker AC300P. Les déplacements chimiques sont exprimés en ppm (partie par million) et les multiplicités par les abréviations usuelles.

### Exemple 1 : Description des protocoles généraux de synthèse des composés décrits

La majeure partie des composés selon l'invention peuvent être notamment obtenus par réduction, selon l'un des protocoles mentionnés ci-dessous, de composés revendiqués et/ou décrits dans la demande de brevet US2005176808.

Les autres composés peuvent être facilement obtenus selon des modes de préparation similaires bien connus et à la portée de l'homme de l'art.

### Procédure générale A : réduction des diphénylpropèn-2-ones par le triéthylsilane

La diphénylpropèn-2-one est solubilisée dans du dichlorométhane. Le triéthylsilane est ajouté puis, goutte à goutte, l'acide trifluoroacétique (7,5 équivalents). Le mélange réactionnel est placé sous agitation à température ambiante et l'avancement de la réaction est suivi par chromatographie sur couche mince. Une fois le produit de départ consommé, le milieu est lavé avec de l'eau. La phase aqueuse est extraite par du dichlorométhane, les phases organiques sont réunies, séchées sur sulfate de magnésium puis le solvant est éliminé par évaporation sous pression réduite. Le résidu d'évaporation est purifié par chromatographie sur gel de silice (HPLC préparative, lichrospher (Merck) RP18 12µm 100Å, colonne : 25*250 mm).

### Procédure générale B : réduction des diphénylpropèn-2-ones par le tétrachlorosilane

La diphénylpropèn-2-one est solubilisée dans de l'acétonitrile. L'iodure de sodium est ajouté puis le tétrachlorosilane goutte à goutte. Le mélange réactionnel est placé sous agitation à température ambiante, l'avancement de la réaction est suivi par chromatographie sur couche mince. Une fois le produit de départ consommé (30 min à 2 heures), le milieu est dilué avec du chloroforme puis lavé avec de l'eau. La phase aqueuse est extraite par du chloroforme, les phases organiques sont réunies et lavées avec une solution saturée de sulfite de sodium puis séchées sur sulfate de magnésium. Le solvant est éliminé par évaporation sous pression réduite. Le résidu d'évaporation est purifié par chromatographie sur gel de silice (HPLC préparative, lichrospher (Merck) RP18 12µm 100Å, colonne : 25*250 mm).

### Procédure générale C : réduction des diphénylpropèn-2-ones par le palladium sur charbon

La diphénylpropèn-2-one est solubilisée dans de l'éthanol puis du palladium sur charbon (10%) en quantité catalytique est ajouté. Le mélange réactionnel est placé sous agitation sous atmosphère d'hydrogène à pression atmosphérique et température ambiante. Une fois le produit de départ consommé, le catalyseur est éliminé par filtration, le solvant est évaporé sous pression réduite et le résidu d'évaporation est purifié par flash chromatographie.

### Procédure générale D : synthèse des alcools

La diphénylpropan-3-one est solubilisée dans de l'éthanol. Le borohydrure de sodium est ajouté. Le mélange réactionnel est maintenu 16 heures sous agitation à 50°C, le milieu est refroidi et hydrolysé. Les solvants sont éliminés par évaporation sous pression réduite, le résidu est repris par une solution aqueuse diluée d'acide chlorhydrique et extrait par du chlorure de méthylène.

La phase organique est lavée avec de l'eau, séchée sur sulfate de magnésium, le chlorure de méthylène est éliminé par évaporation sous pression réduite et le résidu est purifié par chromatographie sur gel de silice (HPLC préparative, lichrospher (Merck) RP18 12µm 100Å, colonne : 25*250 mm).

### Procédure générale E : synthèse des éthers

Le diphénylpropan-3-ol est solubilisé dans un mélange eau/alcool 1/3 :2/3 en présence d'une quantité catalytique d'acide trifluoroacétique. Après 16 h d'agitation à 60°C, le milieu est évaporé sous pression réduite et le résidu d'évaporation est purifié par chromatographie sur gel de silice (HPLC préparative, lichrospher (Merck) RP18 12µm 100Å, colonne : 25*250 mm).

### Procédure générale F : synthèse des oximes et des éthers d'oxime

La diphénylpropan-3-one est solubilisée dans de la pyridine. Le chlorhydrate de O-alkylhydroxylamine est ajouté. Après 16 h de reflux, le milieu est évaporé sous pression réduite et le résidu d'évaporation est purifié par chromatographie flash sur gel de silice.

### Exemple 2 : Synthèse des composés décrits

### Composé 1 de l'invention: Acide 2-[2,6-diméthyl-4-[3-[4-(trifluorométhoxy)phényl]-3-oxo-propyl]phénoxy]-2-méthylpropanoïque

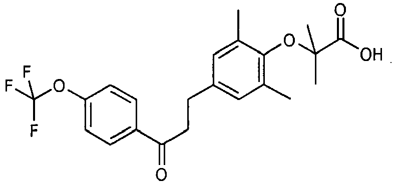

Ce composé a été préparé selon la procédure générale B, à partir de l'acide 2-[2,6-diméthyl-4-[3-[4-(trifluorométhoxy)phényl]-3-oxo-prop-2-ènyl]phénoxy]-2-méthylpropanoïque au moyen de 15 équivalents d'iodure de sodium et 15 équivalents de tétrachlorosilane ;

Aspect : solide blanc ; F= 64-66°C.

RMN ¹H (300 MHz, CDCl₃, δ ppm) : 1,52 (s, 6H), 2,22 (s, 6H), 2,96 (t, 2H, J=7,51Hz), 3,26 (t, 2H, J=7,51 Hz), 6,87 (s, 2H), 7,28 (d, 2H, J=8,61 Hz), 8,01 (d, 2H, J=8,61 Hz). SM(ES-MS) : 423,3 (M-1).

### Composé 2 de l'invention: Acide 2-[2,6-diméthyl-4-[3-[4-(trifluorométhylthio)phény]-3-oxo-propyl]phénoxy]-2-méthylpropanoïque

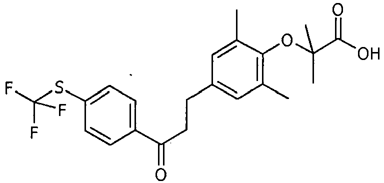

Ce composé a été préparé selon la procédure générale A, à partir de l'acide 2-[2,6-diméthyl-4-[3-[4-(trifluorométhylthio)phényl]-3-oxo-prop-2-ènyl]phénoxy]-2-méthylpropanoïque au moyen de 1 équivalent de triéthylsilane ;

Aspect : solide blanc ; F=83-85 °C.

RMN ¹H (300 MHz, CDCl₃, δ ppm): 1,52 (s, 6H), 2,23 (s, 6H), 2,97 (t, 2H, J=7,59Hz), 3,29 (t, 2H, J=7,59Hz), 6,88 (s, 2H), 7,74 (d, 2H, J=8,46Hz), 7,99 (d, 2H, J=8,46Hz). SM(ES-MS) : 439,2 (M-1).

### Composé 3 de l'invention : Acide 2-[2,6-diméthyl-4-[3-[4-bromophényl]-3-oxopropyl]phénoxy]-2-méthylpropanoïque

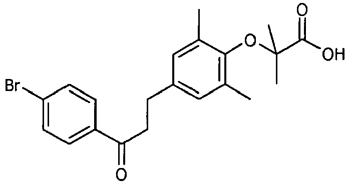

Ce composé a été préparé selon la procédure générale A, à partir de l'acide 2-[2,6-diméthyl-4-[3-[4-bromophényl]-3-oxo-prop-2-ènyl]phénoxy]-2-méthyl- propanoïque au moyen de 1 équivalent de triéthylsilane;

Aspect : huile visqueuse blanche.

RMN ¹H (300 MHz, CDCl₃, δ ppm): 1,52 (s, 6H), 2,23 (s, 6H), 2,96 (t, 2H, J=7,60Hz), 3,24 (t, 2H, J=7,02Hz), 6,89 (s, 2H), 7,61 (d, 2H, J=8,46 Hz), 7,83 (d, 2H, J=8,46Hz). SM(ES-MS) : 417,2 (M-1) ⁷⁹Br et 419,2(M-1) ⁸¹Br.

### Composé 4 de l'invention: Acide 2-[2,6-diméthyl-4-[3-[4-(trifluorométhyl)phényl]-3-oxopropyl]phénoxyl-2-méthylpropanoïque

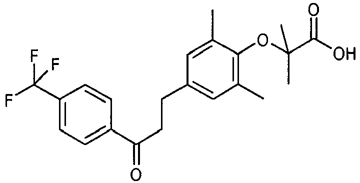

Ce composé a été préparé selon la procédure générale A, à partir de l'acide 2-[2,6-diméthyl-4-[3-[4-(trifluorométhyl)phényl]-3-oxo-prop-2-ènyl]phénoxy]-2-méthylpropanoïque au moyen de 1 équivalent de triéthylsilane ;

Aspect : huile visqueuse jaunâtre.

RMN ¹H (300 MHz, CDCl₃, δ ppm): 1,52 (s, 6H), 2,23 (s, 6H), 2,98 (t, 2H, J=7,29Hz), 3,30 (t, 2H, J=7,29Hz), 6,88 (s, 2H), 7,72 (d, 2H, J=8,17Hz), 8,06 (d, 2H, J=8,17Hz). SM(ES-MS) : 407,4 (M-1).

### Composé 5 : Acide 2-[4-[3-[4-chloro-2-hydroxyphényl]-3-oxo-propyl]phénylthio]-2-méthylpropanoïque

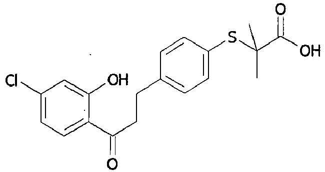

Ce composé a été préparé selon la procédure générale B, à partir de l'acide 2-[4-[3-[4-chloro-2-hydroxyphényl]-3-oxo-prop-2-ènyl]phénylthio]-2-méthyl-propanoïque au moyen de 5 équivalents d'iodure de sodium et 5 équivalents de tétrachlorosilane ;

Aspect : solide blanc ; F=136-137°C.

RMN ¹H (300 MHz, CDCl₃, δ ppm) : 1,52 (s, 6H), 3,08 (t, 2H, J=7,59Hz), 3,30 (t, 2H, J=7,59Hz), 6,87 (dd, 1 H, J=1,89 Hz, J=8,79Hz), 7,02 (d, 1 H, J=1,89Hz), 7,22 (d, 2H, J=7,89Hz), 7,47 (d, 2H, J=8,19Hz), 7,66 (d, 1 H, J=8,46Hz), 12,38 ( s, 1 H). SM(ES-MS) : 377,01 (M-1).

### Composé 6 : Acide 2-[2-méthyl-4-[3-[4-(3,3,3-trifluoropropyloxy)phényl]-3-oxopropyl]phénoxy]-2-méthylpropanoïque

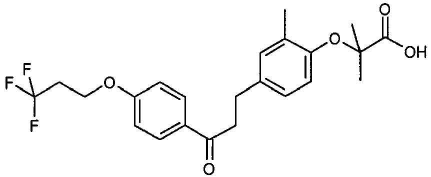

Ce composé a été préparé par réduction selon la procédure générale C du 2-(4-(3-(4-hydroxyphényl)-3-oxo-prop-1-ènyl)-2-méthylphénoxy)-2-méthylpropanoate de tertiobutyle, suivie d'une séquence O-alkylation de phénol / acidolyse d'ester de tertiobutylique selon le brevet US2005176808.

Aspect : huile visqueuse incolore.

RMN ¹H (300 MHz, CDCl₃, δ ppm) : 1,60 (s, 6H); 2,23 (s, 3H); 2,61-2,70 (m, 2H); 2,95-2,99 (m, 2H); 3,19-3,24 (m, 2H); 4,26 (t, 2H, J=6,5Hz); 6,77 (d, 1 H, J=8,5Hz); 6,93 (d, 2H, J=8,9Hz); 6,93-6,98 (m, 1 H); 7,06 (d, 1 H, J=2,1 Hz); 7,95 (d, 2H, J=8,9Hz). SM(ES-MS) : 437,3 (M-1).

### Composé 10 de l'invention : Acide 2-[4-(3-méthoxy-3-(4-(trifluorométhoxy)phényl)propyl)-2,6-diméthylphénoxy]-2-méthylpropanoïque

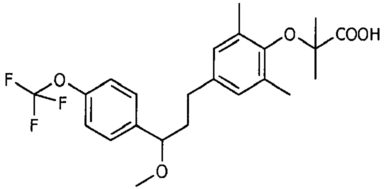

Ce composé a été préparé selon la procédure générale E, à partir d'une solution d'acide 2-(4-(3-hydroxy-3-(4-(trifluorométhoxy)phényl)propyl)-2,6-diméthylphénoxy)-2-méthylpropanoïque dans un mélange eau/méthanol 1/3 :2/3.

Aspect : huile visqueuse incolore.

RMN ¹H (300 MHz, CDCl₃, δ ppm) : 1,46 (s, 6H); 1,91-2,21 (m, 2H); 2,15 (s, 6H); 2,49-2,69 (m, 2H); 3,83 (s, 3H); 4,69 (dd, 1 H, J=7,7Hz J=5,1 Hz); 6,78 (s, 2H) ; 7,19 (d, 2H, J=8,5Hz); 7,36 (d, 2H, J=8,5Hz).

SM(ES-MS) : 458,3 (M+NH4⁺), 463,2 (M+Na⁺), 479,2 (M+K⁺).

### Composé 12 de l'invention : Acide 2-(2,6-diméthyl-4-(3-oxo-3-(4-(2,2,2-trifluoroethoxy)phényl)propyl)phénoxy)-2-méthylpropanoïque

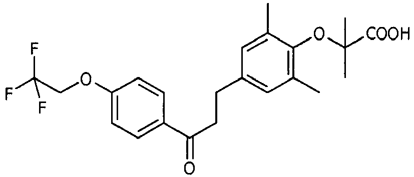

Ce composé a été préparé par réduction selon la procédure générale C du 2-(4-(3-(4-hydroxyphényl)-3-oxo-prop-1-ènyl)-2,6-diméthylphénoxy)-2-méthylpropanoate de tertiobutyle, suivie d'une séquence O-alkylation de phénol / acidolyse d'ester de tertiobutylique selon le brevet US2005176808.

Aspect : solide blanc ; F=98-99°C.

RMN ¹H (300 MHz, CDCl₃, δ ppm) : 1,45 (s, 6H); 2,17 (s, 6H); 2,89 (m, 2H); 3,18 (m, 2H); 4,40 (q, 2H, J=8,1 Hz); 6,82 (s, 2H); 6,95 (d, 2H, J=9,1 Hz); 7,93 (d, 2H, J=9,1 Hz). SM(ES-MS) : 437,4 (M-1).

### Composé 17 : Acide 2-(4-(3-(2-fluoro-4-(2,2,2-trifluoroéthoxy)phényl)-3-oxo-propyl)-2,6-diméthylphénoxy)-2-méthylpropanoïque

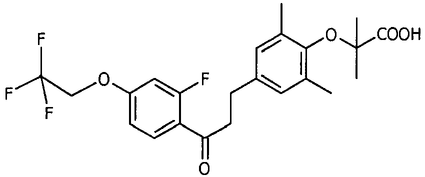

Ce composé a été préparé par réduction selon la procédure générale C du 2-(4-(3-(2-fluoro-4-hydroxyphényl)-3-oxo-prop-1-ènyl)-2,6-diméthylphénoxy)-2-méthylpropanoate de tertiobutyle, suivie d'une séquence O-alkylation de phénol / acidolyse d'ester de tertiobutylique selon le brevet US2005176808.

Aspect : huile visqueuse incolore.

RMN ¹H (300 MHz, CDCl₃, δ ppm) : 1,51 (s, 6H); 2,21 (s, 6H); 2,92 (t, 2H, J=7,6Hz); 3,23 (td, 2H, J=7,6Hz J=3,1 Hz); 6,70 (dd, J=8,7Hz J=2,3Hz); 6,81 (dd, J=12,6Hz J=2,3Hz); 6,86 (s); 7,91 (t, 1 H, J=8,7Hz).

SM(MALDI-TOF) : 479 (M+Na⁺).

### Composé 30 de l'invention : Acide 2-(4-(3-hydroxy-3-(4-(trifluorométhoxy)phényl)propyl)-2,6-diméthylphénoxy)-2-méthylpropanoïque

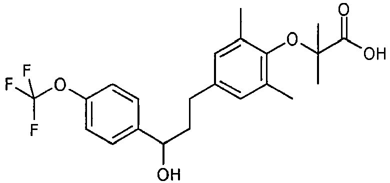

Ce composé a été préparé selon la procédure générale D, à partir de l'acide 2-[2,6-diméthyl-4-[3-[4-(trifluorométhyloxy)phényl]-3-oxo-propyl]phénoxy]-2-méthylpropanoïque au moyen de 4 équivalents de borohydrure de sodium.

Aspect : huile visqueuse incolore.

RMN ¹H (300 MHz, CDCl₃, δ ppm) : 1,46 (s, 6H); 1,94-2,10 (m, 2H); 2,17 (s, 6H); 2,46-2,66 (m, 2H); 4,69 (dd, 1 H, J=7,6Hz J=5,5Hz); 6,78 (s, 2H); 7,17 (d, 2H, J=8,3Hz); 7,34 (d, 2H, J=8,3Hz).

SM(ES-MS) : 425,3 (M-1).

### Composé 33: Acide-2-(4-(3-(méthoxyimino)-3-(4-(trifluorométhoxy)phényl)propyl)-2,6-diméthylphénoxy)-2-méthylpropanoïque

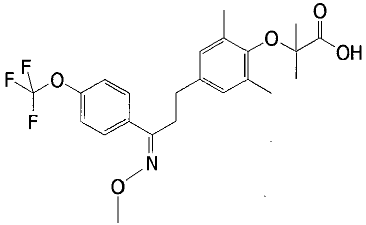

Ce composé a été préparé selon la procédure générale F, à partir de l'acide 2-[2,6-diméthyl-4-[3-[4-(trifluorométhoxy)phényl]-3-oxo-propyl]phénoxy]-2-méthyl-propanoïque. Aspect : huile visqueuse jaunâtre.

RMN ¹H (300 MHz, CDCl₃, δ en ppm) : 1,49 (s, 6H), 2,21 (s, 6H), 2,71-2,77 (m, 2H), 2,95-3,01 (m, 2H), 4,01 (s, 3H), 6,82 (s, 2H), 7,17 (d, 2H, J=8,7Hz), 7,59 (d, 2H, J=8,7Hz).

SM(ES-QTOF) : 476 (M+Na⁺).

### Composé 35 de l'invention : 2-(2,6-diméthyl-4-(3-oxo-3-(4-(trifluorométhoxy)phényl)propyl)phénoxy)-2-méthylpropanoate de tertiobutyle

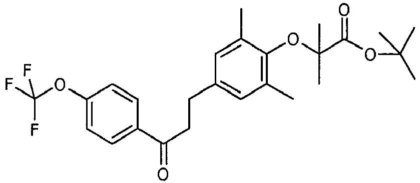

Ce composé a été préparé par réduction selon la procédure générale C de la 3-(4-hydroxy-3,5-diméthylphényl)-1-(4-(trifluorométhoxy)phényl)propan-1-one, suivie d'une séquence O-alkylation de phénol / acidolyse d'ester de tertiobutylique selon le brevet US2005176808.

Aspect : huile visqueuse incolore.

RMN ¹H (300 MHz, CDCl₃, δ ppm) : 1,42 (s, 6H); 1,51 (s, 9H); 2,21 (s, 6H); 2,94 (t, 2H, J=7,9Hz); 3,25 (t, 2H, J=7,9Hz); 6,82 (s, 2H); 7,28 (d, 2H, J=9,1Hz); 8,01 (d, 2H, J=9,1 Hz).

Les autres composés peuvent être obtenus selon des modes de préparation similaires aux procédures générales A à F et à la portée de l'homme de l'art.

### Exemple 3 : Evaluation in vitro des propriétés activatrices des PPARs des composés selon l'invention

Les propriétés activatrices des PPARs des composés selon l'invention sont évaluées *in vitro.*

### Principe

L'activation des PPAR est évaluée *in vitro* sur une lignée de fibroblastes de rein de singe (COS-7) par la mesure de l'activité transcriptionnelle de chimères constituées du domaine de liaison à l'ADN du facteur de transcription Gal4 de la levure et du domaine de liaison au ligand des différents PPAR. Les composés sont testés à des doses comprises entre 10⁻⁷ et 100 µM sur les chimères Gal4-PPAR α, γ, δ.

### Protocole

### Culture des cellules

Les cellules COS-7 proviennent de l'ATCC et sont cultivées dans du milieu DMEM supplémenté de 10% (vol/vol) de sérum de veau foetal, 100 U/ml de pénicilline (Gibco, Paisley, UK) et 2 mM de L-Glutamine (Gibco, Paisley, UK). Les cellules sont incubées à 37°C dans une atmosphère humide contenant 5% de CO₂.

### Description des plasmides utilisés en transfection

Les plasmides Gal4(RE)_TkpGL3, pGal4-hPPARα, pGal4-hPPARγ, pGal4-hPPARδ et pGal4-φ ont été décrits dans la littérature (Raspe E *et al.,* 1999). Les constructions pGal4-hPPARα, pGal4-hPPARγ et pGal4-hPPARδ ont été obtenues par clonage dans le vecteur pGal4-φ de fragments d'ADN amplifiés par PCR correspondants aux domaines DEF des récepteurs nucléaires PPARα, PPARγ et PPARδ humains.

### Transfection

Les cellules COS-7 en suspension sont transfectées avec 150 ng d'ADN par puits, avec un ratio pGal4-PPAR / Gal4(RE)_TkpGL3 de 1/10, en présence de 10% sérum de veau foetal. Les cellules sont ensuite ensemencées dans des plaques de 96 puits (4x10⁴ cellules/puits) puis incubées pendant 24 heures à 37°C. L'activation avec les composés à tester s'effectue pendant 24h à 37°C dans du milieu sans sérum. A l'issue de l'expérience, les cellules sont lysées et l'activité luciférase est déterminée à l'aide du Steady-Lite™ HTS (Perkin Elmer) ou du Steady Glow Luciferase (Promega) selon les recommandations du fournisseur.

### Résultats

Les composés selon l'invention ont été testés sur les 3 isoformes de PPAR. Les résultats obtenus avec les composés 1, 2, 3, 4, 5 et 7 sont détaillés sur les figures (1-1) à (1-18).

Les inventeurs mettent en évidence une augmentation significative et dose-dépendante de l'activité luciférase dans les cellules transfectées avec les plasmides pGal4-hPPAR et traitées avec les composés selon l'invention.

De manière inattendue, les données expérimentales présentées montrent que les composés selon l'invention lient les PPAR *in vitro* et induisent une activation de l'activité transcriptionnelle.

### Exemple 4 : Evaluation in vivo, chez la souris ApoE2/E2, des propriétés sur le poids corporel, des propriétés hypolipémiantes et stimulatrices de la synthèse de HDL-cholestérol des composés selon l'invention

### Principe

Les propriétés sur le poids corporel et les propriétés hypolipémiantes des composés selon l'invention sont évaluées *in vivo* par mesure du poids corporel, par dosage des lipides plasmatiques et par analyse de l'expression génique de gènes cibles des PPAR après traitement de la souris E2/E2 dyslipidémique par les composés selon l'invention.

Le modèle murin utilisé est la souris de type ApoE2/E2, souris transgénique pour l'isoforme E2 de l'apolipoprotéine E humaine (Sullivan PM *et al.,* 1998). Chez l'homme, cette apolipoprotéine, constituant des lipoprotéines de faible et très faible densité (LDL-VLDL), est présente sous trois isoformes E2, E3 et E4. La forme E2 présente une mutation sur un acide aminé en position 158, ce qui affaiblit considérablement l'affinité de cette protéine pour le récepteur aux LDL. La clairance des VLDL est de ce fait quasi nulle. Il se produit alors une accumulation des lipoprotéines de faible densité et une hyperlipidémie mixte dite de type III (cholestérol et triglycérides élevés).

PPARα régule l'expression de gènes impliqués dans le transport des lipides (apolipoprotéines telles que Apo AI, Apo AII et Apo CIII, transporteurs membranaires tels que FAT) ou le catabolisme des lipides (ACO, CPT-I ou CPT-II, enzymes de la β-oxydation des acides gras). Un traitement par les activateurs de PPARα se traduit donc, chez l'homme comme chez le rongeur, par une diminution des taux circulants de triglycérides. La mesure des lipides plasmatiques après traitement par les composés selon l'invention sera donc un indicateur du caractère agoniste de PPAR et donc du caractère hypolipémiant des composés selon l'invention.

Un traitement par les activateurs de PPAR se traduit aussi parfois chez l'homme comme chez le rongeur, par une augmentation du taux de HDL-cholestérol plasmatique. La mesure du taux de HDL-cholesterol plasmatique permet donc de mettre en évidence le caractère stimulateur de la synthèse du HDL-cholestérol des composés selon l'invention.

Les propriétés agonistes de PPARα préalablement mesurées *in vitro* doivent se traduire au niveau hépatique par une sur-expression des gènes cibles directement sous le contrôle du récepteur PPARα : les gènes que nous étudions dans cette expérience sont l'Apo CIII (apolipoprotéine impliquée dans le métabolisme lipidique) et PDK-4 (Pyruvate Deshydrogénase Kinase isoforme 4, enzyme du métabolisme glucidique). La mesure de l'activité transcriptionnelle des gènes cibles de PPARα après traitement par les composés selon l'invention sera donc aussi un indicateur du caractère hypolipémiant des composés selon l'invention.

### Protocole

### Traitement des animaux

Des souris transgéniques Apo E2/E2 ont été maintenues sous un cycle lumière/obscurité de 12/12 heures à une température constante de 20 ± 3°C. Après une acclimatation d'une semaine, les souris ont été pesées et rassemblées par groupes de 6 animaux sélectionnés de telle sorte que la distribution de leurs poids corporels et de leurs taux de lipides plasmatiques déterminés une première fois avant l'expérience soient uniformes. Les composés testés ont été suspendus dans la carboxyméthylcellulose (Sigma C4888) et administrés par gavage intra-gastrique, à raison d'une fois par jour pendant 8 jours à la dose choisie. Les animaux ont eu un accès libre à l'eau et à la nourriture (régime standard). La prise de nourriture et la prise de poids sont enregistrées tout au long de l'expérience. A l'issue de l'expérience, les animaux ont été anesthésiés après un jeûne de 4 heures, un prélèvement sanguin a été effectué sur anticoagulant (EDTA) puis les souris ont été pesées et euthanasiées. Le plasma a été séparé par centrifugation à 3000 tours/minutes pendant 20 minutes, les échantillons ont été conservés à + 4°C.

Des échantillons de foie ont été prélevés et congelés immédiatement dans de l'azote liquide puis conservés à -80°C pour les analyses ultérieures.

### Mesure des lipides plasmatiques

Les concentrations plasmatiques de lipides (cholestérol total et triglycérides) sont mesurées par dosages enzymatiques (bioMérieux-Lyon-France) selon les recommandations du fournisseur.

Les taux de cholestérol et de triglycérides plasmatiques sont mesurés après 8 jours de traitement par voie orale avec les composés selon l'invention ; ces taux sont comparés à ceux obtenus avec des animaux contrôles (non traités par les composés selon l'invention) : la différence mesurée témoigne de l'effet hypolipémiant des composés selon l'invention.

### Mesure du HDL-cholestérol

Les lipoprotéines de basse densité (VLDL et LDL) sont précipitées par Phosphotungstate. Le précipité est éliminé par centrifugation. Le HDL-cholestérol présent dans le surnageant est quantifié par dosages enzymatiques (bioMérieux-Lyon-France) selon les recommandations du fournisseur.

### Analyse d'expression génique par RT-PCR quantitative

L'ARN total est extrait à partir de fragments de foie en utilisant le kit NucleoSpin^{®} 96 RNA (Macherey Nagel, Hoerdt, France) selon les instructions du constructeur.

1 µg d'ARN total (quantifié en utilisant le Ribogreen RNA quantification kit (Molecular Probes)) est ensuite reverse transcrit en ADN complémentaire par une réaction d'1 heure à 37°C dans un volume total de 20 µl contenant du tampon 1X (Sigma), 1,5mM de DTT, 0,18mM de dNTPs (Promega), 200ng de pdN6 (Amersham), 30U d'inhibiteur de RNase (Sigma) et 1 µl de MMLV-RT (Sigma).

Les expériences de PCR quantitative ont été effectuées en utilisant le MyiQ Single-Color Real-Time PCR Detection System (Biorad, Marnes-la-Coquette, France) et ont été réalisées en utilisant le kit iQ SYBR Green Supermix selon les recommandations du fournisseur, en plaques 96 puits, sur 5 µl de réactions de reverse transcription diluées avec une température d'hybridation de 55°C. Des paires d'amorces spécifiques des gènes étudiés ont été utilisées :
- PDK4 : amorce sens : 5'- TACTCCACTGCTCCAACACCTG-3' (SEQ ID NO : 1) et amorce antisens 5'- GTTCTTCGGTTCCCTGCTTG-3' (SEQ ID NO : 2))
- ApoCIII : amorce sens : 5'- CTCTTGGCTCTCCTGGCATC-3' (SEQ ID NO : 3) et amorce antisens 5'- GCATCCTGGACCGTCTTGGA-3' (SEQ ID NO : 4).

La quantité de fluorescence émise est directement proportionnelle à la quantité d'ADN complémentaire présent au début de la réaction et amplifié au cours de la PCR. Pour chaque cible étudiée, une gamme est réalisée par des dilutions successives d'un pool constitué de quelques µl de différentes réactions de reverse transcription. Les niveaux d'expression relatifs de chaque cible sont ainsi déterminés en utilisant les courbes d'efficacité obtenues avec les points de gamme.

Les niveaux d'expression des gènes d'intérêt sont ensuite normalisés par rapport au niveau d'expression du gène de référence 36B4 (dont les amorces spécifiques sont : amorce sens : 5'-CATGCTCAACATCTCCCCCTTCTCC-3' (SEQ ID NO : 7) et amorce antisens : 5'-GGGAAGGTGTAATCCGTCTCCACAG-3' (SEQ ID NO : 8)).

Le facteur d'induction, c'est-à-dire le rapport entre le signal relatif (induit par le composé selon l'invention) et la moyenne des valeurs relatives du groupe contrôle, est ensuite calculé pour chaque échantillon. Plus ce facteur est élevé, plus le composé a un caractère activateur d'expression génique. Le résultat final est représenté comme moyenne des valeurs d'induction dans chaque groupe expérimental.

### Résultats

### Poids corporel

La figure 2-1 compare la prise de poids des animaux après 8 jours de traitement avec le composé 1 administré à 5, 10 et 50 mpk à la prise de poids des animaux contrôle. De manière inattendue, on mesure une perte de poids corporel chez les animaux traités par le composé 1.

### Mesure des lipides plasmatiques

Les figures 2-2 et 2-3 comparent les taux plasmatiques de cholestérol total et de HDL-cholestérol après 8 jours de traitement avec le composé 1 administré à 5, 10 et à 50 mpk aux taux obtenus avec les animaux contrôle. De manière inattendue, les taux circulants de cholestérol total ont été très significativement diminués et les taux de HDL-cholestérol ont été très significativement augmentés par le traitement.

Les figures 2-4 et 2-5 comparent les taux plasmatiques de triglycérides et d'acides gras libres après 8 jours de traitement avec le composé 1 administré à 5, 10 et à 50 mpk aux taux obtenus avec les animaux contrôle. De manière inattendue, les taux circulants de triglycérides et d'acides gras libres ont été très significativement diminués par le traitement.

### Analyse de l'expression génique par RT-PCR quantitative

Les inventeurs ont aussi mis en évidence que les composés selon l'invention sont, *in vivo,* des régulateurs de l'expression de gènes cibles des PPARs. Les résultats présentés sur les figures 2-6 et 2-7 montrent que le composé 1 administré à 5, 10 et à 50 mpk pendant 8 jours à des souris E2/E2, induit une augmentation significative de l'expression hépatique des gènes codant pour PDK4 (figure 2-6) et une diminution de l'expression hépatique du gène codant pour ApoCIII (figure 2-7). L'ensemble de ces gènes codent pour des enzymes fortement impliquées dans le métabolisme des lipides et des glucides et le fait que leur expression soit modulée par les composés selon l'invention renforce l'idée que ces composés présentent un intérêt potentiel majeur dans le cadre des pathologies métaboliques.

### Conclusion

De manière inattendue, les données expérimentales présentées montrent que les composés selon l'invention induisent *in vivo* une perte de poids corporel, stimulent la synthèse de HDL-cholestérol en parallèle d'un effet hypolipémiant (diminution des taux plasmatiques de cholesterol total, de triglycérides et d'acides gras libres). De plus, les données expérimentales présentées montrent que les composés selon l'invention modulent l'expression de gènes régulés par l'activation des PPARs qui codent pour des enzymes fortement impliquées dans le métabolisme des lipides et des glucides.

### Exemple 5 : Evaluation in vivo, chez la souris C57BI6, des propriétés sur le poids corporel, des propriétés hypolipémiantes et stimulatrices de la synthèse de HDL-cholestérol des composés selon l'invention.

### Principe

Les propriétés sur le poids corporel et les propriétés hypolipémiantes des composés selon l'invention sont évaluées *in vivo* par mesure du poids corporel, par dosage des lipides plasmatiques et par analyse de l'expression génique de gènes cibles des PPARs après traitement par voie orale, par les composés selon l'invention, de la souris C57B16.

### Protocole

### Traitement des animaux

Des souris C57BI6 femelles ont été maintenues sous un cycle lumière/obscurité de 12/12 heures à une température constante de 20 ± 3°C. Après une acclimatation d'une semaine, les souris ont été pesées et rassemblées par groupes de 6 animaux sélectionnés de telle sorte que la distribution de leurs poids corporel et de leurs taux de lipides plasmatiques déterminés une première fois avant l'expérience soient uniformes. Les composés testés ont été suspendus dans la carboxyméthylcellulose (Sigma C4888) et administrés par gavage intra-gastrique, à raison d'une fois par jour pendant 14 jours à la dose choisie. Les animaux ont eu un accès libre à l'eau et à la nourriture (régime standard). La prise de nourriture et la prise de poids sont enregistrées tout au long de l'expérience. A l'issue de l'expérience, les animaux ont été anesthésiés après un jeûne de 4 heures, un prélèvement sanguin a été effectué sur anticoagulant (EDTA) puis les souris ont été pesées et euthanasiées. Le plasma a été séparé par centrifugation à 3000 tours/minutes pendant 20 minutes, les échantillons ont été conservés à + 4°C. Des échantillons de tissu hépatique ont été prélevés et congelés immédiatement dans de l'azote liquide puis conservés à -80°C pour les analyses ultérieures.

### Mesure du HDL-cholestérol

Les lipoprotéines de basse densité (VLDL et LDL) sont précipitées par Phosphotungstate. Le précipité est éliminé par centrifugation. Le HDL-cholestérol présent dans le surnageant est quantifié par dosages enzymatiques (bioMérieux-Lyon-France) selon les recommandations du fournisseur.

### Mesure des triglycérides plasmatiques

Les concentrations plasmatiques de triglycérides ont été mesurées par dosages enzymatiques (bioMérieux-Lyon-France) selon les recommandations du fournisseur.

### Analyse d'expression génique par RT-PCR quantitative

L'ARN total a été extrait à partir de fragments de foie en utilisant le kit NucleoSpin^{®} 96 RNA (Macherey Nagel, Hoerdt, France) selon les instructions du fabricant.

1 µg d'ARN total (quantifié par spectrophotométrie) a ensuite été reverse transcrit en ADN complémentaire par une réaction d'1 heure à 37°C dans un volume total de 20 µl contenant du tampon 1X (Sigma), 1,5mM de DTT, 0,18mM de dNTPs (Promega), 200ng de pdN6 (Amersham), 30U d'inhibiteur de RNase (Sigma) et 1 µl de MMLV-RT (Sigma).

Les expériences de PCR quantitative ont été effectuées en utilisant le MyiQ Single-Color Real-Time PCR Detection System (Biorad, Marnes-la-Coquette, France) et ont été réalisées en utilisant le kit iQ SYBR Green Supermix selon les recommandations du fournisseur, en plaques 96 puits, sur 5 µl de réaction de reverse transcription diluée avec une température d'hybridation de 55°C. Des paires d'amorces spécifiques des étudiés ont été utilisées.
- PDK4 : amorce sens : 5'- TACTCCACTGCTCCAACACCTG-3' (SEQ ID NO : 1) et amorce antisens 5'- GTTCTTCGGTTCCCTGCTTG-3' (SEQ ID NO : 2))
- ApoCIII: amorce sens : 5'- CTCTTGGCTCTCCTGGCATC-3' (SEQ ID NO : 3) et amorce antisens 5'- GCATCCTGGACCGTCTTGGA-3' (SEQ ID NO : 4).

La quantité de fluorescence émise est directement proportionnelle à la quantité d'ADN complémentaire présent au début de la réaction et amplifié au cours de la PCR. Pour chaque cible étudiée, une gamme est réalisée par des dilutions successives d'un pool constitué de quelques microlitres de différentes réactions de reverse transcription. Les niveaux d'expression relatifs de chaque cible sont ainsi déterminés en utilisant les courbes d'efficacité obtenues avec les points de gamme.

Les niveaux d'expression des gènes d'intérêt ont ensuite été normalisés par rapport au niveau d'expression du gène de référence 36B4 (dont les amorces spécifiques sont : amorce sens : 5'-CATGCTCAACATCTCCCCCTTCTCC-3' (SEQ ID NO : 7) et amorce antisens : 5'-GGGAAGGTGTAATCCGTCTCCACAG-3' (SEQ ID NO : 8)).

Le facteur d'induction a ensuite été calculé pour chaque échantillon. Plus ce facteur est élevé, plus le composé a un caractère activateur d'expression génique. Le résultat final est représenté comme moyenne des valeurs d'induction dans chaque groupe expérimental.

### Résultats

### Poids corporel

La figure 3-1 compare la prise de poids des animaux après 14 jours de traitement avec le composé 1 administré à 3, 10 et 30 mpk à la prise de poids des animaux contrôle. De manière inattendue, on mesure une perte de poids corporel chez les animaux traités par le composé 1.

### Mesure des lipides plasmatiques

La figure 3-2 compare les taux plasmatiques de HDL-cholestérol après 14 jours de traitement avec le composé 1 administré à 3, 10 et 30 mpk aux taux obtenus avec les animaux contrôle. De manière inattendue, les taux circulants de HDL-cholestérol ont été très significativement augmentés par le traitement.

La figure 3-3 compare les taux plasmatiques de triglycérides après 14 jours de traitement avec le composé 1 administré à 3, 10 et 30 mpk aux taux obtenus avec les animaux contrôle. De manière inattendue, les taux circulants de triglycérides ont été très significativement diminués par le traitement.

### Analyse de l'expression génique par RT-PCR quantitative

Les inventeurs ont aussi mis en évidence que les composés selon l'invention sont, *in vivo,* des régulateurs de l'expression de gènes cibles des PPARs. Les résultats présentés sur les figures 3-4 et 3-5 montrent que le composé 1 administré à 3, 10 et à 30 mpk pendant 14 jours à des souris C57BI6, induit une augmentation significative de l'expression hépatique des gènes codant pour PDK4 (figure 3-4) et une diminution de l'expression hépatique du gène codant pour ApoCIII (figure 3-5). L'ensemble de ces gènes codent pour des enzymes fortement impliquées dans le métabolisme des lipides et des glucides et le fait que leur expression soit modulée par les composés selon l'invention renforce l'idée que ces composés présentent un intérêt potentiel majeur dans le cadre des pathologies métaboliques.

### Conclusion

De manière inattendue, les données expérimentales présentées montrent que les composés selon l'invention induisent *in vivo* une perte de poids corporel, stimulent la synthèse de HDL-cholestérol en parallèle d'un effet hypolipémiant (diminution des taux plasmatiques de triglycérides). De plus, les données expérimentales présentées montrent que les composés selon l'invention modulent l'expression de gènes régulés par l'activation des PPARs qui codent pour des enzymes fortement impliquées dans le métabolisme des lipides et des glucides.

### Exemple 6 : Evaluation in vivo, chez la souris db/db, des propriétés sur le poids corporel, des propriétés antidiabétique, hypolipémiantes et stimulatrices de la synthèse de HDL-cholestérol des composés selon l'invention.

### Principe

Les propriétés sur le poids corporel, l'insulino-resistance et les propriétés hypolipémiantes des composés selon l'invention sont évaluées *in vivo* par mesure du poids corporel, par mesure des taux plasmatiques de glucose et d'insuline, par dosage des lipides plasmatiques, analyse de la répartition du cholestérol dans les différentes fractions lipoprotéiques plasmatiques et par analyse de l'expression génique de gènes cibles des PPAR après traitement par voie orale, par les composés selon l'invention, de la souris db/db.

### Protocole

### Traitement des animaux

Des souris db/db femelles ont été maintenues sous un cycle lumière/obscurité de 12/12 heures à une température constante de 20 ± 3°C. Après une acclimatation d'une semaine, les souris ont été pesées et rassemblées par groupes de 8 animaux sélectionnés de telle sorte que la distribution de leurs poids corporel et de leurs taux de lipides plasmatiques déterminés une première fois avant l'expérience soient uniformes. Les composés testés ont été suspendus dans la carboxyméthylcellulose (Sigma C4888) et administrés par gavage intra-gastrique, à raison d'une fois par jour pendant 28 jours à la dose choisie. Les animaux ont eu un accès libre à l'eau et à la nourriture (régime standard). La prise de nourriture et la prise de poids sont enregistrées tout au long de l'expérience. A l'issue de l'expérience, les animaux ont été anesthésiés après un jeûne de 4 heures, un prélèvement sanguin a été effectué sur anticoagulant (EDTA) puis les souris ont été pesées et euthanasiées. Le plasma a été séparé par centrifugation à 3000 tours/minutes pendant 20 minutes, les échantillons ont été conservés à + 4°C. Des échantillons de tissu hépatique et de tissu musculaire squelettique ont été prélevés et congelés immédiatement dans de l'azote liquide puis conservés à -80°C pour les analyses ultérieures.

### Mesure de la glycémie et de l'insulinémie plasmatique

Le glucose plasmatique murin est dosé 'selon une méthode enzymo-colorimétrique utilisant le kit Glucose RTU (Biomérieux). Le glucose est transformé en acide gluconique par action de la glucose oxydase et cette réaction libère du péroxyde d'hydrogène. Le péroxyde d'hydrogène est dosé selon la réaction de Trinder qui, par action d'une peroxydase en présence de phénol et de amino-4-antipyrine, produit de l'eau et un produit coloré, la quinonéimine. L'intensité de la coloration due à la quinonéimine est proportionnelle à la quantité de glucose présente dans l'échantillon.

Le dosage de l'insuline murine est effectué par la méthode Elisa (en utilisant le kit INSKR020 du fournisseur Crystal chem). Un anticorps anti-insuline de souris est coaté sur une microplaque. Le sérum à doser pour l'insuline est ensuite déposé sur cette plaque. Un anticorps anti-insuline de cobaye va reconnaître le complexe insuline/anticorps monoclonal anti-insuline de souris et s'y fixer. Enfin un anticorps anti-cobaye marqué à la péroxydase est ajouté se fixant à l'anticorps anti-insuline de cobaye. La réaction colorimétrique est réalisée par addition du substrat de l'enzyme OPD (Ortho Phényl Diamine). L'intensité de la coloration est proportionnelle à la quantité d'insuline présente dans l'échantillon.

### Mesure des lipides plasmatiques,

Les concentrations plasmatiques de lipides (cholestérol total et triglycérides) sont mesurées par dosages enzymatiques (bioMérieux-Lyon-France) selon les recommandations du fournisseur.

### Analyse de la répartition du cholestérol dans les fractions lipoprotéiques plasmatiques.

Les différentes fractions lipidiques (VLDL, LDL, HDL) du plasma ont été séparées par une chromatographie Gel - Filtration. Les concentrations de cholestérol ont ensuite été mesurées dans chaque fraction par dosages enzymatiques (bioMérieux-Lyon-France) selon les recommandations du fournisseur.

### Analyse d'expression génique par RT-PCR quantitative

### Tissu hépatique

L'ARN total a été extrait à partir de fragments de foie en utilisant le kit NucleoSpin® 96 RNA (Macherey Nagel, Hoerdt, France) selon les instructions du fabricant.

### Tissu squelettique

L'ARN total a été extrait à partir de fragments de muscle squelettique gastrocnémien en utilisant le kit RNeasy^{®} Fibrous Tissue kit (Qiagen) selon les instructions du fabricant.

1 µg d'ARN total (quantifié par spectrophotométrie) a ensuite été reverse transcrit en ADN complémentaire par une réaction d'1 heure à 37°C dans un volume total de 20 µl contenant du tampon 1X (Sigma), 1,5mM de DTT, 0,18mM de dNTPs (Promega), 200ng de pdN6 (Amersham), 30U d'inhibiteur de RNase (Sigma) et 1 µl de MMLV-RT (Sigma).

Les expériences de PCR quantitative ont été effectuées en utilisant le MyiQ Single-Color Real-Time PCR Detection System (Biorad, Marnes-la-Coquette, France) et ont été réalisées en utilisant le kit iQ SYBR Green Supermix selon les recommandations du fournisseur, en plaques 96 puits, sur 5 µl de réaction de reverse transcription diluée avec une température d'hybridation de 55°C. Des paires d'amorces spécifiques des étudiés ont été utilisées.
- PDK4 : amorce sens : 5'- TACTCCACTGCTCCAACACCTG-3' (SEQ ID NO : 1) et amorce antisens 5'- GTTCTTCGGTTCCCTGCTTG-3' (SEQ ID NO : 2))
- UCP2: amorce sens: 5'- GTCGGAGATACCAGAGCACTGTCG -3' (SEQ ID NO : 5) et amorce antisens 5'- CACATCAACAGGGGAGGCGA -3' (SEQ ID NO : 6)

La quantité de fluorescence émise est directement proportionnelle à la quantité d'ADN complémentaire présent au début de la réaction et amplifié au cours de la PCR. Pour chaque cible étudiée, une gamme est réalisée par des dilutions successives d'un pool constitué de quelques microlitres de différentes réactions de reverse transcription. Les niveaux d'expression relatifs de chaque cible sont ainsi déterminés en utilisant les courbes d'efficacité obtenues avec les points de gamme.

Les niveaux d'expression des gènes d'intérêt ont ensuite été normalisés, dans le tissu hépatique par rapport au niveau d'expression du gène de référence 36B4 (dont les amorces spécifiques sont : amorce sens : 5'-CATGCTCAACATCTCCCCCTTCTCC-3' (SEQ ID NO : 7) et amorce antisens : 5'-GGGAAGGTGTAATCCGTCTCCACAG-3' (SEQ ID NO : 8)) et, dans le tissu musculaire squelettique par rapport au niveau d'expression du gène de référence 18S (dont les amorces spécifiques sont : amorce sens : 5'-CGGACACGGACAGGATTGACAG-3' (SEQ ID NO : 9) et l'amorce antisens : 5'-AATCTCGGGTGGCTGAACGC-3' (SEQ ID NO: 10)). Le facteur d'induction, a ensuite été calculé pour chaque échantillon. Plus ce facteur est élevé, plus le composé a un caractère activateur d'expression génique. Le résultat final est représenté comme moyenne des valeurs d'induction dans chaque groupe expérimental.

### Résultats

### Poids corporel

La figure 4-1 compare la prise de poids des animaux après 28 jours de traitement avec le composé 1 administré à 50 mpk à la prise de poids des animaux contrôle. De manière inattendue, on mesure une perte de poids corporel chez les animaux traités par le composé 1.

### Mesure de la glycémie et de l'insulinémie

Les figures 4-2 et 4-3 comparent les taux plasmatiques de glucose et d'insuline après 28 jours de traitement avec le composé 1 administré à 50 mpk. De manière inattendue, la glycémie et l'insulinémie sont significativement diminués par le traitement.

### Mesure des lipides plasmatiques

La figure 4-4 compare les taux plasmatiques de cholestérol total après 28 jours de traitement avec les composés 1 et 3 administrés à 50 mpk aux taux obtenus avec les animaux contrôle. De manière inattendue les taux de cholestérol total ont été significativement augmentés. La figure 4-5 montre que cette augmentation du cholestérol total plasmatique correspond à une augmentation significative de la fraction HDL-cholestérol induite par le traitement des animaux avec les composés 1 et 3 à 50 mpk.

Les figures 4-6 et 4-7 comparent les taux plasmatiques de triglycérides et d'acides gras libres après 28 jours de traitement avec les composés 1 et 3 administrés à 50 mpk aux taux obtenus avec les animaux contrôle. De manière inattendue, les taux circulants de triglycérides et d'acides gras libres ont été très significativement diminués par les traitements.

### Analyse de l'expression génique par RT-PCR quantitative

Les inventeurs ont aussi mis en évidence que les composés selon l'invention sont, *in vivo,* des régulateurs de l'expression de gènes cibles des PPARs. Les résultats présentés sur les figures 4-8 et 4-9 montrent que les composés 1 et 3 administrés à 50 mpk pendant 28 jours à des souris db/db, induisent une augmentation significative de l'expression hépatique du gènes codant pour PDK4 (figure 4-8) et une diminution de l'expression dans le muscle squelettique du gène codant pour UCP2 (figure 4-9). L'ensemble de ces gènes codent pour des enzymes fortement impliquées dans le métabolisme des lipides, des glucides et la dissipation d'énergie et le fait que leur expression soit modulée par les composés selon l'invention renforce l'idée que ces composés présentent un intérêt potentiel majeur dans le cadre des pathologies métaboliques.

### Conclusion

De manière inattendue, les données expérimentales présentées montrent que les composés selon l'invention induisent *in vivo* une perte de poids corporel, une amélioration de la sensibilité à l'insuline, stimulent la synthèse de HDL-cholestérol en parallèle d'un effet hypolipémiant (diminution des taux plasmatiques de triglycérides). De plus, les données expérimentales présentées montrent que les composés selon l'invention modulent l'expression de gènes régulés par l'activation des PPARs qui codent pour des enzymes fortement impliquées dans le métabolisme des lipides, des glucides et la dissipation d'énergie.

### Exemple 7 : Evaluation in vitro des propriétés anti-inflammatoires des composés selon l'invention

### Principe

Les effets anti-inflammatoires des composés selon l'invention ont été évalués par la mesure de la sécrétion de MCP1 (Monocyte chemotactic protein-1) par des monocytes traités pendant 24 heures avec les composés selon l'invention et simultanément stimulés avec du PMA (Phorbol 12-myristate 13-acetate, provoque une réponse inflammatoire des cellules et leur différentiation en macrophages). Plus la quantité de MCP1 sécrétée est diminuée, plus le composé selon l'invention inhibe la réaction inflammatoire.

### Protocole

### Culture et traitement des cellules THP-1.

La lignée de monocytes humains THP1 (provenance ATCC) est cultivée dans du milieu RPMI1640 additionné de 25mM Hepes (Gibco ; 42401-018), 1% glutamine (Gibco ; 25030-24) 1 % pénicilline/streptomycine (Biochrom AG ; A 2213) et 10% sérum de veau foetal décomplémenté (SVF. Gibco ; 26050-088).

Les cellules ensemencées sur des plaques de 24 puits (Primaria BD Falcon) à la densité de 870000 cellules/puit puis sont incubées à 37°C et 5% de CO₂ pendant 24h dans du milieu de culture contenant 0.2% de sérum de veau foetal en présence de 5 ng/ml de phorbol 12-myristate 13-acetate (PMA) et 1µM du composé 3 selon l'invention. Le composé selon l'invention est dissous dans du diméthyl sulfoxide (DMSO, Fluka ; 41640). L'effet des composés selon l'invention est comparé à l'effet du DMSO seul.

### Mesure de la sécrétion de MCP1

Le milieu de traitement est récupéré et la concentration de MCP1 est mesurée en utilisant la trousse Elisa «Human MCP-1 ELISA Set » (BD OptEIA ; 555179) selon les recommandations du fabricant.

MCP1 est fixé sur une plaque et reconnue par un anticorps spécifique anti-MCP1. L'anticorps est, à son tour, spécifiquement reconnu par un deuxième type d'anticorps couplé à une enzyme peroxydase. La coloration résultant de l'activité enzymatique est proportionnelle à la quantité d'MCP1 fixée et peut être mesurée par spectrophotométrie. Une gamme est réalisée à partir d'un point de concentration connue et permet de calculer la concentration en MCP1 de chaque échantillon.

Le facteur d'induction, c'est-à-dire le rapport entre le signal induit par le composé selon l'invention et le signal du groupe contrôle, a ensuite été calculé. Plus ce facteur est faible, plus le composé a un caractère inhibiteur de la sécrétion d'MCP1. Le résultat final est représenté comme moyenne des valeurs d'induction de chaque groupe expérimental.

### Résultats

Les inventeurs ont mis en évidence, sur des monocytes *in vitro,* que les composés selon l'invention ont des effets anti-inflammatoires. Les résultats présentés sur la figure 5 montrent que le composé 3 selon l'invention, à 1µM, induit une diminution significative de la sécrétion de MCP1 par les monocytes.

### Conclusion

De manière inattendue, les données expérimentales présentées montrent que les composés selon l'invention ont une action anti-inflammatoire dans des monocytes stimulés au PMA

### Conclusion générale

Les inventeurs ont mis en évidence que les composés selon l'invention entrainent une perte de poids corporel, ont des propriétés hypolipémiantes, en baissant les taux de cholestérol et de triglycérides plasmatiques, des propriétés stimulatrices de la synthèse de HDL-cholestérol ainsi que des propriétés antidiabétiques. De plus, les inventeurs ont mis en évidence que les composés selon l'invention sont des régulateurs de l'expression de gènes codant pour des enzymes fortement impliquées dans le métabolisme des lipides, des glucides et la dissipation d'énergie.

Les inventeurs ont également mis en évidence que les composés selon l'invention présentaient des propriétés anti-inflammatoire.

Ces résultats, obtenus *in vivo* et *in vitro* témoignent du potentiel thérapeutique des composés selon l'invention vis-à-vis de pathologies majeures telles que les dyslipidémies, le diabète de type 2 et l'obésité.

### BIBLIOGRAPHIE

Fox-Tucker J, The Cardiovasular Market Outlook to 2010, BUSINESS INSIGHTS REPORTS, 2005, 1-174

Gross B, et al., Peroxisome Proliferator-Activated Receptor b/d: A novel target for the reduction of atherosclerosis, DRUG DISCOVERY TODAY: THERAPEUTIC STRATEGIES, 2005, 2 (3), 237-243

International Atherosclerosis Society, Harmonized Clinical. Guidelines on Prevention of Atherosclerotic Vascular Disease, 2003,

Kota BP, et al., An overview on biological mechanisms of PPAR, Pharmacol Res, 2005, 51 (2), 85-94

Lefebvre P, et al., Sorting out the roles of PPARalpha in energy metabolism and vascular homeostasis, J Clin Invest, 2006, 116 (3), 571-580

Lehrke M and Lazar MA, The many faces of PPARgamma, Cell, 2005, 123 (6), 993-9

Liu Y and Miller A, Ligands to peroxisome proliferator-activated receptors as therapeutic options for metabolic syndrome, DRUG DISCOVERY TODAY: THERAPEUTIC STRATEGIES, 2005, 2 (3), 165-169

Mensah M, The Atlas of Heart Disease and Stroke, 2004,

Raspe E, et al., Modulation of rat liver apolipoprotein gene expression and serum lipid levels by tetradecylthioacetic acid (TTA) via PPAR{alpha} activation, J. Lipid Res., 1999, 40 (11), 2099-2110

Sullivan PM, et al., Type III hyperlipoproteinemia and spontaneous atherosclerosis in mice resulting from gene replacement of mouse Apoe with human Apoe*2, J Clin Invest, 1998, 102 (1), 130-5.

## Revendications

1. Composés dérivés de 1,3-diphénylpropane substitués de formule générale (I) : 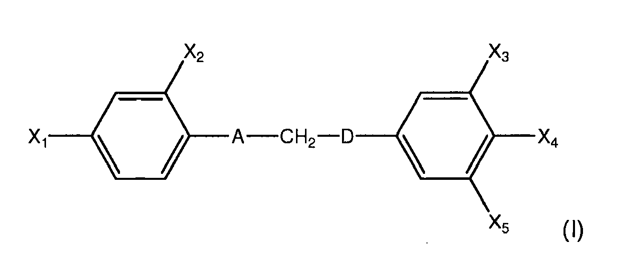 dans laquelle :
X1 représente un atome d'halogène, un groupement R1 ou G1-R1 ;
X2 représente un atome d'hydrogène ;
X3 représente un groupement R3 ;
X4 représente G4-R4, dans lequel G4 représente un atome d'oxygène ;
X5 représente un groupement R5 ;
R1 représentant un groupement alkyle halogéné ;
R3, et R5, identiques ou différents, représentant un groupement alkyle non substitué;
G1 représentant un atome d'oxygène ou de soufre ;
R4 représentent un groupement alkyle substitué par un groupement COOR9 ;
A représente
(i) un groupement -CR6R7 dans lequel :
R6 représente un atome d'hydrogène,
R7 représente un groupement hydroxy ou un groupement -ORB, R8 représentant un groupement alkyle, substitué ou non par un groupement aryle hétéroaryle ou cycloalkyle ;
ou
(ii) un groupement carbonyle,
D représente un atome de carbone lié à deux atomes d'hydrogène,
R9 représentant un atome d'hydrogène ou un radical alkyle non substitué ;
leurs stéréoisomères, diastéréoisomères, énantiomères, purs ou en mélange, mélanges racémiques, isomères géométriques, tautomères, sels, hydrates, solvates, formes solides ainsi que leurs mélanges.

2. Composés selon la revendication 1, **caractérisés en ce que** A représente un groupement carbonyle (CO).

3. Composés selon la revendication 1 ou 2, **caractérisés en ce que** X4 représente un groupement G4R4, dans lequel G4 représente un atome d'oxygène et R4 représente un représente un groupement alkyle comprenant 1-10 atomes de carbone et substitué par un groupement -COOR9, R9 étant tel que défini dans la revendication 1.

4. Composés selon la revendication 3, **caractérisés en ce que** le groupement X4 répond à la formule -OC(CH₃)₂COOR9, R9 étant tel que défini dans la revendication 1.

5. Composés selon l'une quelconque des revendications précédentes, **caractérisés en ce que** R3 et R5 représentent un groupement alkyle non substitué comprenant 1, 2, 3 ou 4 atomes de carbone.

6. Composés selon l'une quelconque des revendications précédentes, **caractérisés en ce que** X1 représente un atome d'halogène ou un groupement G1R1, G1 étant tel que défini dans la revendication 1 et R1 représentant un groupement alkyle halogéné comportant 1, 2, ou 3 atomes de carbone.

7. Composés selon l'une quelconque des revendications précédentes, **caractérisés en ce qu'**ils sont choisis parmi :
Acide 2-[2,6-diméthyl-4-[3-[4-(trifluorométhoxy)phényl]-3-oxo-propyl]phénoxy]-2-méthylpropanoïque,
Acide 2-[2,6-diméthyl-4-[3-[4-(trifluorométhylthio)phényl]-3-oxo-propyl]phénoxy]-2-méthylpropanoïque,
Acide 2-[2,6-diméthyl-4-[3-[4-bromophényl]-3-oxo-propyl]phénoxy]-2-méthylpropanoïque,
Acide 2-[2,6-diméthyl-4-[3-[4-(trifluorométhyl)phényl]-3-oxo-propyl]phénoxy]-2-méthylpropanoïque,
Acide 2-[2,6-diméthyl-4-[3-hydroxy-3-[4-(trifluorométhylthio)phényl]propyl]phénoxy]-2-méthylpropanoïque,
Acide 2-[2,6-diméthyl-4-(3-(pyridin-3-ylméthoxy)-3-(4-(trifluorométhoxy)phényl)propyl)phénoxy]-2-méthylpropanoïque,
Acide 2-[4-(3-(4-iodobenzyloxy)-3-(4-(trifluorométhoxy)phényl)propyl)-2,6-diméthylphénoxy]-2-méthylpropanoïque,
Acide 2-[4-(3-méthoxy-3-(4-(trifluorométhoxy)phényl)propyl)-2,6-diméthylphénoxy]-2-méthylpropanoïque,
Acide 2-[2,6-diméthyl-4-[3-[4-(3,3,3-trifluoropropyloxy)phényl]-3-oxopropyl]phénoxy]-2-méthylpropanoïque,
Acide 2-(2,6-diméthyl-4-(3-oxo-3-(4-(2,2,2-trifluoroéthoxy)phényl)propyl)phénoxy)-2-méthylpropanoïque,
Acide 2-(2,6-diméthyl-4-(3-oxo-3-(4-(2,2,2-trifluoroéthylthio)phényl)propyl) phénoxy)-2-méthylpropanoïque,
Acide 2-(2,6-diméthyl-4-(3-oxo-3-(4-(trifluorométhoxy)phényl)propyl)phénoxy) propano'ique,
Acide 2-(4-(3-hydroxy-3-(4-(trifluorométhoxy)phényl)propyl)-2,6-diméthylphénoxy)-2-méthylpropanoïque,
Acide 4-(2,6-diméthyl-4-(3-oxo-3-(4-(trifluorométhoxy)phényl)propyl)phénoxy)-2,2-diméthylbutanoïque,
2-(2,6-diméthyl-4-(3-oxo-3-(4-(trifluorométhoxy)phényl)propyl)phénoxy)-2-méthylpropanoate de tertiobutyle,
2-(2,6-diméthyl-4-(3-oxo-3-(4-(trifluorométhoxy)phényl)propyl)phénoxy)-2-méthylpropanoate d'isopropyle.

8. Composition pharmaceutique comprenant, dans un support acceptable sur le plan pharmaceutique, au moins un composé tel que défini dans l'une des revendications 1 à 7, éventuellement en association avec un ou plusieurs autres principes actifs thérapeutiques et/ou cosmétiques.

9. Composition pharmaceutique selon la revendication 8, pour le traitement des complications associées au syndrome métabolique, de l'insulino-résistance, du diabète, des dyslipidémies, de l'athérosclérose, des maladies cardiovasculaires, de l'obésité, de l'hypertension, des maladies inflammatoires, des pathologies neurodégénératives, ou des cancers.

10. Composition pharmaceutique selon la revendication 8, pour le traitement des dyslipidémies.

11. Composition pharmaceutique selon la revendication 8, pour traiter les facteurs de risque cardiovasculaire liés aux dérèglements du métabolisme lipidique et/ou glucidique.

## Patentansprüche

1. Verbindungen substituierter 1,3-Diphenylpropanderivate der allgemeinen Formel (I): 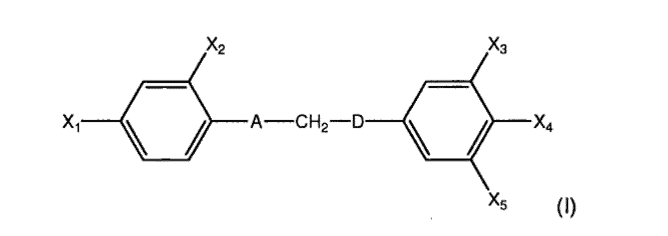 in der:
X1 für ein Halogenatom, eine Gruppe R1 oder G1-R1 steht;
X2 für ein Wasserstoffatom steht;
X3 für eine Gruppe R3 steht;
X4 für G4-R4 steht, wobei G4 für ein Sauerstoffatom steht;
X5 für eine Gruppe R5 steht;
wobei R1 für eine halogenierte Alkyl-Gruppe steht;
wobei R3 und R5, die identisch oder verschieden sind, für eine unsubstituierte Alkyl-Gruppe stehen;
wobei G1 für ein Sauerstoff- oder Schwefelatom steht;
wobei R4 für eine Alkyl-Gruppe steht, die mit einer Gruppe COOR9 substituiert ist;
A für
(i) eine Gruppe -CR6R7 steht, in der:
R6 für ein Wasserstoffatom steht,
R7 für eine Hydroxy-Gruppe oder -OR8-Gruppe steht, wobei R8 für eine Alkyl-Gruppe steht,
die mit einer Aryl-, Heteroaryl- oder Cycloalkyl-Gruppe substituiert sein kann;
oder
(ii) eine Carbonyl-Gruppe steht,
D für ein Kohlenstoffatom steht, das mit zwei Wasserstoffatomen verbunden ist,
wobei R9 für ein Wasserstoffatom oder einen unsubstituierten Alkyl-Rest steht;
ihre Stereoisomere, Diastereoisomere, Enantiomere, rein oder als Gemisch, racemischen Gemische, geometrischen Isomere, Tautomere, Salze, Hydrate, Solvate, festen Formen sowie ihre Gemische.

2. Verbindungen gemäß Anspruch 1, **dadurch gekennzeichnet, dass** A für eine Carbonyl-(CO)-Gruppe steht.

3. Verbindungen gemäß Anspruch 1 oder 2, **dadurch gekennzeichnet, dass** X4 für eine Gruppe G4R4 steht, in der G4 für ein Sauerstoffatom steht und R4 für einen Vertreter einer Alkyl-Gruppe steht, die 1-10 Kohlenstoffatome umfasst und mit einer Gruppe -COOR9 substituiert ist, wobei R9 wie in Anspruch 1 definiert ist.

4. Verbindungen gemäß Anspruch 3, **dadurch gekennzeichnet, dass** die Gruppe X4 der Formel - OC(CH₃)₂COOR9 entspricht, wobei R9 wie in Anspruch 1 definiert ist.

5. Verbindungen gemäß einem der vorhergehenden Ansprüche, **dadurch gekennzeichnet, dass** R3 und R5 für eine unsubstituierte Alkyl-Gruppe stehen, die 1, 2, 3 oder 4 Kohlenstoffatome umfasst.

6. Verbindungen gemäß einem der vorhergehenden Ansprüche, **dadurch gekennzeichnet, dass** X1 für ein Halogenatom oder eine Gruppe G1R1 steht, wobei G1 wie in Anspruch 1 definiert ist und R1 für eine halogenierte Alkyl-Gruppe steht, die 1, 2 oder 3 Kohlenstoffatome umfasst.

7. Verbindungen gemäß einem der vorhergehenden Ansprüche, **dadurch gekennzeichnet, dass** sie ausgewählt sind aus:
2-[2,6-Dimethyl-4-[3-[4-(trifluormethoxy)phenyl]-3-oxo-propyl]phenoxy]-2-methylpropansäure,
2-[2,6-Dimethyl-4-[3-[4-(trifluormethylthio)phenyl]-3-oxo-propyl]phenoxy]-2-methylpropansäure,
2-[2,6-Dimethyl-4-[3-[4-bromphenyl]-3-oxo-propyl]phenoxy]-2-methylpropansäure,
2-[2,6-Dimethyl-4-[3-[4-(trifluormethyl)phenyl]-3-oxo-propyl]phenoxy]-2-methylpropansäure,
2-[2,6-Dimethyl-4-[3-hydroxy-3-[4-(trifluormethylthio)phenyl]propyl]phenoxy]-2-methylpropansäure,
2-[2,6-Dimethyl-4-(3-(pyridin-3-ylmethoxy)-3-(4-(trifluormethoxy)phenyl)propyl)phenoxy]-2-methylpropansäure,
2-[4-(3-(4-Iodbenzyloxy)-3-(4-(trifluormethoxy)phenyl)propyl)-2,6-dimethylphenoxy]-2-methylpropansäure,
2-[4-(3-Methoxy-3-(4-(trifluormethoxy)phenyl)propyl)-2,6-dimethylphenoxy]-2-methylpropansäure,
2-[2,6-Dimethyl-4-[3-[4-(3,3,3-trifluorpropyloxy)phenyl]-3-oxo-propyl]phenoxy]-2-methylpropansäure,
2-(2,6-Dimethyl-4-(3-oxo-3-(4-(2,2,2-trifluorethoxy)phenyl)propyl)phenoxy)-2-methylpropansäure,
2-(2,6-Dimethyl-4-(3-oxo-3-(4-(2,2,2-trifluorethylthio)phenyl)propyl)phenoxy)-2-methylpropansäure,
2-(2,6-Dimethyl-4-(3-oxo-3-(4-(trifluormethoxy)phenyl)propyl)phenoxy)propansäure,
2-(4-(3-Hydroxy-3-(4-(trifluormethoxy)phenyl)propyl)-2,6-dimethylphenoxy)-2-methylpropansäure,
4-(2,6-Dimethyl-4-(3-oxo-3-(4-(trifluormethoxy)phenyl)propyl)phenoxy)-2,2-dimethylbutansäure,
2-(2,6-Dimethyl-4-(3-oxo-3-(4-(trifluormethoxy)phenyl)propyl)phenoxy)-2-methylpropansäure-tertiobutylester,
2-(2,6-Dimethyl-4-(3-oxo-3-(4-(trifluormethoxy)phenyl)propyl)phenoxy)-2-methylpropansäure-isopropylester.

8. Pharmazeutische Zusammensetzung, umfassend in einem pharmazeutisch geeigneten Träger wenigstens eine wie in einem der Ansprüche 1 bis 7 definierte Verbindung, eventuell in Kombination mit einem oder mehreren anderen therapeutischen und/oder kosmetischen Wirkstoffen.

9. Pharmazeutische Zusammensetzung gemäß Anspruch 8 zur Behandlung von Komplikationen, die mit dem metabolischen Syndrom, der Insulinresistenz, Diabetes, Dyslipidämien, der Arteriosklerose, kardiovaskulären Erkrankungen, der Obesität, der Hypertonie, Entzündungskrankheiten, neurodegenerativen Erkrankungen oder Krebsen assozüert sind.

10. Pharmazeutische Zusammensetzung gemäß Anspruch 8 zur Behandlung von Dyslipidämien.

11. Pharmazeutische Zusammensetzung gemäß Anspruch 8 zur Behandlung von kardiovaskulären Risikofaktoren, die mit Störungen des Lipid- und/oder Kohlenhydratstoffwechsels verbunden sind.

## Claims

1. Compounds, derived from substituted 1,3-diphenylpropane, having the general formula (I): 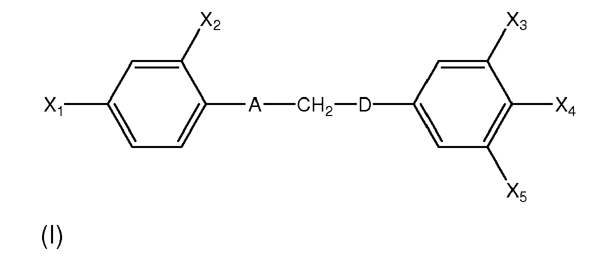 in which:
X1 represents a halogen atom, a R1 or G1-R1 group;
X2 represents a hydrogen atom;
X3 represents a R3 group;
X4 represents a G4-R4 group, wherein R4 represents an oxygen atom;
X5 represents a R5 group;
R1 representing a halogenated alkyl group;
R3 and R5, identical or different, representing an unsubstituted alkyl group;
G1 representing an atom of oxygen or sulfur;
R4 represents an alkyl group substituted by a COOR9 group;
A represents:
(i) a -CR6R7 group, in which:
R6 represents a hydrogen atom,
R7 represents an hydroxy group or a -OR8 group, R8 representing an alkyl group, substituted or not by an aryl, heteroaryl or cycloalkyl group, or
(ii) a carbonyl group (CO),
D represents a carbon atom linked to two hydrogen atoms (CH₂),
R9 representing an atom of hydrogen or an unsubstituted alkyl radical;
their stereoisomers, diastereoisomers, enantiomers, pure or mixed, racemic mixtures, geometrical isomers, tautomers, salts, hydrates, solvates, solid forms as well as their mixtures.

2. Compounds according to claim 1, **characterized in that** A represents a carbonyl group (CO).

3. Compounds according to claim 1 or 2, **characterized in that** X4 represents G4R4, in which G4 represents an oxygen atom and R4 represents an alkyl group comprising 1 to 10 carbon atoms and substituted by a COOR9 group, R9 being as defined in claim 1.

4. Compounds according to claim 3, **characterized in that** X4 corresponds to the formula -OC(CH₃)₂COOR9, R9 being as defined in claim 1.

5. Compounds according to any one of the previous claims, **characterized in that** R3 and R5 represent an unsubstituted alkyl group comprising 1, 2, 3, or 4 carbon atoms.

6. Compounds according to any one of the previous claims, **characterized in that** X1 represents a halogen atom or G1 R1 group, G1 as defined in claim 1 and R1 representing a halogenated alkyl group comprising 1, 2, or 3 carbon atoms.

7. Compounds according to any one of the previous claims, **characterized in that** they are selected from:
2-[2,6-dimethyl-4-[3-[4-(trifluoromethoxy)phenyl]-3-oxo-propyl]phenoxy]-2-methylpropanoic acid,
2-[2,6-dimethyl-4-[3-[4-(trifluoromethylthio)phenyl]-3-oxo-propyl]phenoxy]-2-methylpropanoic acid,
2-[2,6-dimethyl-4-[3-[4-bromophenyl]-3-oxo-propyl]phenoxy]-2-methylpropanoic acid,
2-[2,6-dimethyl-4-[3-[4-(trifluoromethyl)phenyl]-3-oxo-propyl]phenoxy]-2-methylpropanoic acid,
2-[2,6-dimethyl-4-[3-hydroxy-3-[4-(trifluoromethylthio)phenyl]propyl]phenoxy]-2-methylpropanoic acid,
2-[2,6-dimethyl-4-(3-(pyridin-3-ylmethoxy)-3-[4-(trifluoromethoxy)phenyl]propyl]phenoxy]-2-methylpropanoic acid,
2-[4-(3-(4-iodobenzyloxy)-3-(4-(trifluoromethoxy)phenyl)propyl)-2,6-dim6thylph6noxy]-2-methylpropanoic acid,
2-[4-(3-(4-methoxy)-3-(4-(trifluoromethoxy)phenyl)propyl)-2,6-dimethylphenoxy]-2-methylpropanoic acid,
2-[2,6-di methyl-4-[3-[4-(3,3,3-trifluoropropyloxy)phenyl]-3-oxo-propyl]phenoxy]-2-methylpropanoic acid,
2-(2,6-dimethyl-4-(3-oxo-3-(4-(2,2,2-trifluoroethoxy)phenyl)propyl)phenoxy)-2-methylpropanoic acid,
2-(2,6-dimethyl-4-(3-oxo-3-(4-(2,2,2-trifluoroethylthio)phenyl)propyl)phenoxy)-2-methylpropanoic acid,
2-(2,6-dimethyl-4-(3-oxo-3-(4-(trifluoromethoxy)phenyl)propyl)phenoxy)propanoic acid,
2-[4-(3-hydroxy-3-(4-(trifluoromethoxy)phenyl)propyl)-2,6-dimethylphenoxy]-2-methylpropanoic acid,
4-(2,6-dimethyl-4-(3-oxo-3-(4-(trifluoromethoxy)phenyl)propyl)phenoxy)-2,2-dimethylbutanoic acid,
2-(2,6-dimethyl-4-(3-oxo-3-(4-(trifluoromethoxy)phenyl)propyl)phenoxy)-2-tertiobutyl methylpropanoate,
2-(2,6-dimethyl-4-(3-oxo-3-(4-(trifluoromethoxy)phenyl)propyl)phenoxy)-2-isopropyl methylpropanoate.

8. A pharmaceutical composition comprising, in a pharmaceutically acceptable support, at least one compound as defined in anyone of claims 1 to 7, possibly in association with one or several other therapeutic and/or cosmetic active constituents.

9. A pharmaceutical composition according to claim 8, for the treatment of complications associated with metabolic syndrome, insulin resistance, diabetes, dyslipidemias, atherosclerosis, cardiovascular diseases, obesity, hypertension, inflammatory diseases, neurodegenerative pathologies, or cancers.

10. A pharmaceutical composition according to claim 8 for the treatment of dyslipidemias.

11. A pharmaceutical composition according to claim 8 for treating cardiovascular risk factors relative to a deregulation of lipid and/or glucid metabolism.
